# EUROPEAN PATENT APPLICATION

(11) **EP 4 343 002 A1**
(43) Date of publication of application: **27.03.2024**
(21) Application number: 22804054.9
(22) Date of filing: 20.05.2022
(51) Int. Cl.: C12Q 1/6883, C12Q 1/6813

(54) **COMPOSITION AND METHOD FOR ANALYZING TARGET MOLECULE FROM SAMPLE**

(30) Priority: 21.05.2021 CN 202110557216
(71) Applicant: 10K Genomics, Shanghai 200233 (CN)
(72) Inventor: SHI, Weiyang, Shanghai 200233 (CN)
(74) Representative: Weickmann & Weickmann PartmbB
(86) International application number: PCT/CN2022/094017
(87) International publication number: WO 2022/242734

(57) **Abstract**

A composition and method for analyzing a target molecule from a sample. The method comprises: contacting at least one target molecule in a sample with at least one probe, the probe comprising a target molecule binding domain; attaching the probe to a sample-specific barcode, so as to obtain a barcoded probe; and determining the composition of the barcoded probe, and determining the presence and/or content of the target molecule in the sample on the composition of the barcoded probe.

## Description

### Field of the Invention

The present application relates to the field of biomedicine, and in particular to a composition and method for analyzing a target molecule from a sample.

### Background of the Invention

At present, nucleic acid sequencing technology has undergone rapid and tremendous progress. Sequencing technology produces a large number of sequence data, which can be used to study and interpret genomes and genome regions, and provide information widely used in routine biological research and diagnosis.

Traditional research methods for genome or transcriptome expression are usually carried out at multicellular level. Therefore, the final signal value is the average signal from multiple cells, and the information of cell heterogeneity is lost. Therefore, characteristic signals from certain cell types in complex tissues (e.g., brain) can only be studied by individually analyzing single cells.

At present, when conducting sequencing analysis in single-cell omics (such as transcriptome), it is generally necessary to amplify the target nucleic acid through a reverse transcription step before sequencing. There are many problems in this method. On the one hand, long nucleic acid molecules (such as cDNA molecules obtained after reverse transcription) need to be sequenced to determine their corresponding gene expression products, which is slow, inefficient and costly. On the other hand, due to the potential loss of information or systematic bias in the reverse transcription process, the detection accuracy will be low. In addition, this method cannot be used for simultaneous high-throughput analysis of multiple omics of single cells.

Therefore, there is an urgent need for a new method in this field, which can perform high-throughput analysis of transcriptomics information or other omics information of single cells efficiently, accurately, quickly and inexpensively.

### Summary of the invention

The present application provides a method for analyzing a target molecule from a sample having one or more of the following properties: 1) hybridization of a probe with a target molecule in the sample (e.g., the target molecule may involve a variety of omics, such as transcriptome, proteome, etc.) without the need for reverse transcription and with a high efficiency of gene detection; 2) sequencing only the probe itself, thus reducing the sequencing cost; 3) it is possible to design probes and sequence specifically targeting genes of interest; 4) suitable for clinical sample research.

In one aspect, the present application provides a method for analyzing a target molecule from a sample, the method including:
contacting at least one target molecule in the sample with at least one probe, which contains a target molecule binding domain;
attaching the probe to a sample-specific barcode to obtain a barcoded probe; and
determining the composition of the barcoded probe, and determining the presence and/or content of the target molecule in the sample from the composition of the barcoded probe.

In some embodiments, the target molecule includes a protein, a nucleic acid molecule, a metabolite and/or a lipid.

In some embodiments, the nucleic acid molecule includes a DNA molecule and/or an RNA molecule.

In some embodiments, the DNA includes cDNA.

In some embodiments, the RNA includes cRNA, mRNA, miRNA, IncRNA, and/or eRNA.

In some embodiments, the sample includes at least one cell.

In some embodiments, the sample is a single cell.

In some embodiments, the target molecule in the sample is the presence and/or content of one or more mRNA in a single cell.

In some embodiments, the cell is selected from the group consisting of a cell isolated from an organism, a cell cultured *in vitro,* a primary cell, and a discrete cell from an explant.

In some embodiments, the cell is immobilized.

In some embodiments, the target molecule includes a nucleic acid molecule, and the target molecule binding domain in the probe includes a single-stranded nucleic acid.

In some embodiments, the single-stranded nucleic acid includes at least 10 nucleotides.

In some embodiments, the single-stranded nucleic acid includes about 10-60 nucleotides.

In some embodiments, the target molecule binding domain in the probe is at least 50% complementary to the sequence of the target molecule.

In some embodiments, the composition of the target molecule is known.

In some embodiments, the target molecule is mRNA and the nucleic acid sequence of the mRNA is known.

In some embodiments, the target molecule binding domain in the probe is designed according to the nucleic acid sequence of the mRNA.

In some embodiments, the probe further includes a probe identity tag.

In some embodiments, in the probe, the target molecule binding domain is directly or indirectly linked with the probe identity tag.

In some embodiments, the probe identity tag includes a unique molecular identification region.

In some embodiments, the unique molecular identification region includes a single-stranded nucleic acid.

In some embodiments, the single-stranded nucleic acid includes 1-20 nucleotides.

In some embodiments, the target molecule binding domain is directly or indirectly linked to the antigen binding portion.

In some embodiments, the method includes contacting the at least one target molecule in the sample with two or more probes.

In some embodiments, the two or more probes have respective distinct probe identity tags.

In some embodiments, the method includes contacting at least one target molecule in the sample with the first probe and contacting the target molecule with a second probe different from the first probe.

In some embodiments, a 3' end of the first probe is linked directly or indirectly to a 5' end of the second probe.

In some embodiments, a 5' end of the first probe is linked directly or indirectly to a 3' end of the second probe.

In some embodiments, the 3' end of the first probe is linked to the 5' end of the second probe by a ligase.

In some embodiments, the 5' end of the first probe is linked to the 3' end of the second probe by a ligase.

In some embodiments, the first probe and the second probe form a single-stranded cyclic molecule.

In some embodiments, a nucleic acid sequence at the 3' end of the first probe is complementary to a nucleic acid sequence at the 3' end of the second probe, and a nucleic acid sequence of the first probe is complementary to a nucleic acid sequence at the 5' end of the second probe.

In some embodiments, the 5' end of the second probe is linked directly or indirectly to the 3' end of the second probe.

In some embodiments, the 5' end of the second probe is linked to the 3' end of the second probe by a ligase.

In some embodiments, the ligase includes a T4 ligase.

In some embodiments, in the method, the probes in contact with target molecules derived from the same sample are attached with the same sample-specific barcode.

In some embodiments, the target molecule in the sample is mRNA in a single cell and the method does not include a reverse transcription step.

In some embodiments, the probe further includes an amplification primer binding region.

In some embodiments, the probe further includes an oligonucleotide adapter.

In some embodiments, the amplification primer binding region in the probe is directly or indirectly linked to the probe identity tag.

In some embodiments, the amplification primer binding region in the probe is directly linked to the probe identity tag.

In some embodiments, at least two probes of the two or more probes are capable of binding to different regions of the same target molecule in the sample.

In some embodiments, the method includes, after contacting the target molecule in the sample with the probe and before attaching the probe with the sample-specific barcode, providing a solid support attached with at least one PCR tag, wherein each of the PCR tags contains a sample-specific barcode and a PCR handle sequence at the 3' end of the sample-specific barcode which is a PCR handle sequence the same as the 5' end sequence of the probe, and providing a free primer which is complementary to the sequence at the 3' end of the probe.

In some embodiments, the method further includes co-dispensing the probe, the solid support attached with at least one PCR tag, and the primer into a discrete compartment, and attaching the probe with the sample-specific barcode.

In some embodiments, the PCR tag is releasably attached to the solid support.

In some embodiments, the method includes releasing the at least one PCR tag from the solid support and co-dispensing the PCR tag, the primer, and the probe into the discrete compartment, enabling the probe to be attached with the sample-specific barcode.

In some embodiments, the PCR tag is directly or indirectly attached to the solid support through its 5' end.

In some embodiments, a PCR reagent is further included in the discrete compartment, and under the action of the PCR reagent, the probe is attached with a sample-specific barcode so as to obtain a barcoded probe.

In some embodiments, the method includes co-dispensing the probe and two or more of the PCR tags, the probe, and the primer into the discrete compartment, and the two or more PCR tags are different, such that the barcoded probe includes two or more sample-specific barcodes.

In some embodiments, the method includes co-dispensing the probe and three or more of the PCR tags, the probe, and the primer into the discrete compartment, and the three or more PCR tags are different, such that the barcoded probe includes three or more sample-specific barcodes.

In some embodiments, in the barcoded probe, the probe is located at the 3' end of the sample specific barcode.

In some embodiments, the solid support is a bead.

In some embodiments, the bead is a magnetic bead.

In some embodiments, the discrete compartments are pores or microdroplets.

In some embodiments, the sample-specific barcode includes a cell barcode, and the cell barcodes included in each PCR tag attached to the same solid support are the same.

In some embodiments, the cell barcode includes at least two cell barcode segments spaced by a linker sequence.

In some embodiments, the method includes making the primer complementary to the sequence at the 3' end of the probe and extending to obtain a sequence complementary to the sequence at the 5' end of the probe.

In some embodiments, the method includes hybridizing a sequence complementary to a sequence at the 5' end of the probe with a PCR handle sequence in the PCR tag and attaching the probe with a sample-specific barcode to obtain a barcoded probe.

In some embodiments, the PCR tag further includes an amplification primer recognition region.

In some embodiments, the amplification primer recognition region is a universal amplification primer recognition region.

In some embodiments, the barcoded probe in the method includes the sample-specific barcode and the probe.

In some embodiments, the sample-specific barcode is located at the 3' end of the probe in the method.

In some embodiments, the method further includes, after obtaining the barcoded probe and before determining the composition of the barcoded probe, releasing the barcoded probe from the discrete compartment.

In some embodiments, the method includes, after releasing the barcoded probe from the discrete compartment and before determining the composition of the barcoded probe, sequencing the barcoded probe to determine the composition of the barcoded probe.

In some embodiments, the method includes, after determining the composition of the barcoded probes, determining the presence and/or content of the target molecule from the number of the barcoded probes.

In some embodiments, the method includes, after determining the composition of the barcoded probe, determining the presence and/or content of the target molecule from the number of probe identification tags in the barcoded probe.

In some embodiments, the method includes, after contacting the target molecule in the sample with the probe and before attaching the probe with the sample-specific barcode, providing a solid support attached with at least one oligonucleotide tag, wherein each of the oligonucleotide tags includes a sample-specific barcode and a hybridization sequence at the 3' end of the sample-specific barcode.

In some embodiments, the probe includes a first strand and a second strand, and the first strand includes a target molecule binding domain.

In some embodiments, the second strand includes a second portion complementary to the oligonucleotide adapter in the probe, and the second strand includes a first portion complementary to the hybridization sequence at the 3' end of the sample-specific barcode in the oligonucleotide tag, the first and second strands forming a partially double-stranded structure.

In some embodiments, the probe is linked to the solid support attached with at least one oligonucleotide tag in the discrete section to obtain the barcoded probe.

In some embodiments, the oligonucleotide tag is releasably attached to the solid support.

In some embodiments, the method includes releasing the at least one oligonucleotide tag from the solid support and attaching to a first strand in the probe, enabling the probe to be attached with a sample-specific barcode.

In some embodiments, the oligonucleotide tag is directly or indirectly attached to the solid support through its 5' end.

In some embodiments, a ligase is further included in the discrete compartment, and the ligase enables the oligonucleotide tag to be linked with a first strand in the probe.

In some embodiments, the ligase includes a T4 ligase.

In some embodiments, in the barcoded probe, the probe is located at the 3' end of the sample-specific barcode.

In some embodiments, the solid support is a bead.

In some embodiments, the bead is a magnetic bead.

In some embodiments, the discrete compartments are pores or microdroplets.

In some embodiments, the sample-specific barcode in the method includes a cell barcode, and the cell barcodes included in each oligonucleotide tag attached to the same solid support are the same.

In some embodiments, the cell barcode includes at least two cell barcode segments spaced by a linker sequence.

In some embodiments, the method includes linking the hybridization sequence in the oligonucleotide tag to a first strand in the probe to produce the barcoded probe.

In some embodiments, the method includes hybridizing the first portion of a second strand in the probe with the hybridization sequence of the oligonucleotide tag and linking the hybridization sequence of the oligonucleotide tag with the first strand in the probe to produce the barcoded probe.

In some embodiments, the oligonucleotide tag further includes an amplification primer recognition region.

In some embodiments, the amplification primer recognition region in the method is a universal amplification primer recognition region.

In some embodiments, the barcoded probe includes the sample-specific barcode and the probe.

In some embodiments, the sample-specific barcode in the method is located at the 3' end of the probe.

In some embodiments, the method further includes, after obtaining the barcoded probe and before determining the composition of the barcoded probe, releasing the barcoded probe from the discrete compartment.

In some embodiments, the method includes, after releasing the barcoded probe from the discrete compartment and before determining the composition of the barcoded probe, sequencing the barcoded probe to determine the composition of the barcoded probe.

In some embodiments, the method includes, after determining the composition of the barcoded probes, determining the presence and/or content of the target molecule from the number of barcoded probes.

In some embodiments, the method includes, after determining the composition of the barcoded probe, determining the presence and/or content of the target molecule from the number of probe identity tags in the barcoded probe.

In some embodiments, in the method, after contacting the target molecule in the sample with the probe and before attaching the probe with the sample-specific barcode, providing the solid support attached with at least one oligonucleotide tag, wherein each of the oligonucleotide tags comprises the first strand and the second strand, and the first strand includes the sample-specific barcode and the hybridization sequence at the 3' end of the sample-specific barcode, the second strand includes the first portion complementary to the hybridization sequence of the first strand and the second portion complementary to the sequence of the oligonucleotide adapter in the probe, and the first and second strands forming a partially double-stranded structure, and the solid support attached with at least one oligonucleotide tag is linked with the probe in the discrete section to obtain a barcoded probe.

In some embodiments, the oligonucleotide tag in the method is releasable attached to the solid support.

In some embodiments, the method includes releasing the at least one oligonucleotide tag from the solid support and linking with the probe to attach the probe with the sample-specific barcode.

In some embodiments, the oligonucleotide tag is directly or indirectly attached to the solid support through the 5' end of its first strand.

In some embodiments, a ligase is further included in the discrete compartment, and the ligase enables the oligonucleotide tag to be linked with the probe.

In some embodiments, the ligase includes a T4 ligase.

In some embodiments, in the barcoded probe, the probe is located at the 3' end of the sample-specific barcode.

In some embodiments, the solid support is a bead.

In some embodiments, the bead is a magnetic bead.

In some embodiments, the discrete compartments are pores or microdroplets.

In some embodiments, the sample-specific barcode includes a cell barcode, and the cell barcodes included in each oligonucleotide tag attached to the same solid support are the same.

In some embodiments, the cell barcode includes at least two cell barcode segments spaced by a linker sequence.

In some embodiments, the method includes linking the hybridization sequence of a first strand of the oligonucleotide tag to the probe to produce the barcoded probe.

In some embodiments, the method includes hybridizing the second portion of the second strand of the oligonucleotide tag to the oligonucleotide adapter in the probe, and linking the hybridization sequence of the first strand of the oligonucleotide tag to the probe to produce the barcoded probe.

In some embodiments, the oligonucleotide tag in the method further includes amplification primer recognition regions.

In some embodiments, the amplification primer recognition region in the method is a universal amplification primer recognition region.

In some embodiments, the barcoded probe in the method includes the sample-specific barcode and the probe.

In some embodiments, the sample-specific barcode in the method is located at the 3' end of the probe in the method.

In some embodiments, the method further includes, after obtaining the barcoded probe and before determining the composition of the barcoded probe, releasing the barcoded probe from the discrete compartment.

In some embodiments, the method includes, after releasing the barcoded probe from the discrete compartment and before determining the composition of the barcoded probe, sequencing the barcoded probe to determine the composition of the barcoded probe.

In some embodiments, the method includes, after determining the composition of the barcoded probe, determining the presence and/or content of the target molecule from the number of barcoded probes.

In some embodiments, the method includes after determining the composition of the barcoded probe, determining the presence and/or content of the target molecule from the number of probe identification tags in the barcoded probe.

In some embodiments, the target molecule includes a protein, and the target molecule binding domain in the probe includes an antigen binding portion that specifically recognizes the protein.

In some embodiments, the antigen binding portion includes an antigen binding protein and/or an antigen binding nucleic acid molecule.

In some embodiments, the antigen binding protein includes an antibody or an antigen binding fragment.

In some embodiments, the antigen binding fragment includes Fab, Fab', F(ab)2, Fv fragments, F(ab')2, scFv, di-scFv, VHH, and/or dAb.

In some embodiments, the antigen binding nucleic acid molecule includes a nucleic acid aptamer.

In some embodiments, the identity of the protein is known.

In some embodiments, the amino acid sequence of the protein is known.

In some embodiments, the amino acid sequence of the antibody or antigen binding fragment is known.

In some embodiments, the probe further includes a probe identity tag.

In some embodiments, the target molecule binding domain is directly or indirectly linked to the probe identity tag.

In some embodiments, the probe identity tag characterizes the identity of the antibody or antigen binding fragment.

In some embodiments, the method includes contacting the target molecule in the sample with two or more probes.

In some embodiments, the two or more probes in the method have respective different probe identity tags.

In some embodiments, in the method, the probes in contact with target molecules derived from the same sample are attached with the same sample-specific barcode.

In some embodiments, the probe further includes an amplification primer binding region.

In some embodiments, the probe further includes an oligonucleotide adapter.

In some embodiments, the amplification primer binding region in the probe is directly or indirectly linked to the probe identity tag.

In some embodiments, the amplification primer binding region in the probe is directly linked to the probe identity tag.

In some embodiments, the method provides after contacting the target molecule in the sample with the probe and before attaching the probe with the sample-specific barcode, providing a solid support attached with at least one oligonucleotide tag, wherein each of the oligonucleotide tags includes the first strand and the second strand, and the first strand includes the sample-specific barcode and the hybridization sequence at the 3' end of the sample-specific barcode, the second strand comprises the first portion complementary to the hybridization sequence of the first strand and the second portion complementary to the sequence of the oligonucleotide adapter in the probe, and the first and second strands forming a partially double-stranded structure, and the solid support attached with at least one oligonucleotide tag is linked with the probe in the discrete compartment to obtain a barcoded probe.

In some embodiments, the oligonucleotide tag is releasably attached to the solid support.

In some embodiments, the method includes releasing the at least one oligonucleotide tag from the solid support and linking with the probe to attach the probe with the sample-specific barcode.

In some embodiments, the oligonucleotide tag is directly or indirectly attached to the solid support through the 5' end of its first strand.

In some embodiments, a ligase is further included in the discrete compartment, and the ligase enables the oligonucleotide tag to be linked with the probe.

In some embodiments, the ligase includes a T4 ligase.

In some embodiments, in the barcoded probe, the probe is located at the 3' end of the sample-specific barcode.

In some embodiments, the solid support in the method is a bead.

In some embodiments, the bead is a magnetic bead.

In some embodiments, the discrete compartments are pores or microdroplets.

In some embodiments, the sample-specific barcode includes cell barcodes, and the cell barcodes included in each oligonucleotide tag attached to the same solid support are the same.

In some embodiments, the cell barcode includes at least two cell barcode segments spaced by a linker sequence.

In some embodiments, the method includes linking the hybridization sequence of a first strand of the oligonucleotide tag to the probe to produce the barcoded probe.

In some embodiments, the method includes hybridizing the second portion of the second strand of the oligonucleotide tag to the oligonucleotide adapter in the probe, and linking the hybridization sequence of the first strand of the oligonucleotide tag to the probe to produce the barcoded probe.

In some embodiments, the oligonucleotide tag further includes an amplification primer recognition region.

In some embodiments, the amplification primer recognition region is a universal amplification primer recognition region.

In some embodiments, the barcoded probe includes the sample-specific barcode and the probe.

In some embodiments, in the method, the sample-specific barcode is located at the 3' end of the probe.

In some embodiments, the method further includes, after obtaining the barcoded probe and before determining the composition of barcoded probe, releasing the barcoded probe from the discrete compartment.

In some embodiments, the method includes, after determining the composition of the barcoded probes, determining the presence and/or content of the target molecule from the number of barcoded probes.

In some embodiments, the method includes, after determining the composition of the barcoded probe, determining the presence and/or content of the target molecule from the number of probe identification tags in the barcoded probe.

In some embodiments, the method includes, before contacting the target molecule in a sample with a probe, pretreating the sample.

In some embodiments, the pretreatment includes immobilizing the sample with an immobilizing agent selected from one or more of the group consisting of formaldehyde, paraformaldehyde, methanol, ethanol, acetone, glutaraldehyde, osmium acid and potassium dichromate.

In some embodiments, the pretreatment includes exposing the nucleus of the sample.

In some embodiments, the pretreatment includes treating the sample with a detergent including Triton, NP-40, and/or digitonin.

In some embodiments, the target molecule includes protein and mRNA.

In some embodiments, the target molecule binding domain specifically recognizes the protein and binds mRNA.

In another aspect, the present application provides a combination including:
1) two or more probes including a target molecule binding domain and a probe identity tag;
2) a plurality of solid supports, each of the solid supports being attached with at least one PCR tag, where each of the PCR tags includes a sample-specific barcode and a PCR handle sequence at the 3' end of the sample-specific barcode, the PCR handle sequence being same to the sequence at the 5' end of the probe and providing a free primer and a PCR reagent, the primer being complementary to the sequence at the 3' end of the probe.

In some embodiments, the 1) and 2) exist independently of each other.

In some embodiments, the 1) and 2) exist as a mixture.

In another aspect, the present application provides a kit for analyzing a sample including the combination.

In some embodiments, the kit includes at least one of a nucleic acid amplifying agent, an immobilizing agent, a permeating agent, and a lysing agent.

In some embodiments, the kit contains an instruction for use.

In some embodiments, the instruction for use includes the following steps:
contacting at least one target molecule in a sample with at least one probe, the probe including a target molecule binding domain and a probe identity tag;
attaching the probe to a sample-specific barcode to obtain a barcoded probe; and
determining the composition of the barcoded probe, and determining the presence and/or content of the target molecule in the sample from the composition of the barcoded probe.

In another aspect, the present application provides a combination including:
1) two or more probes including a target molecule binding domain and a probe identity tag;
2) a plurality of solid supports, each of the solid supports being attached with at least one oligonucleotide tag, where each of the oligonucleotide tags includes a first strand and a second strand, the first strand including a sample-specific barcode and a hybridization sequence at a 3' end of the sample-specific barcode, the second strand including a first portion complementary to a hybridization sequence of the first strand and a second portion complementary to the probe identity tag, and the first and second strands forming a partially double-stranded structure.

In some embodiments, the 1) and 2) exist independently of each other.

In some embodiments, the 1) and 2) exist as a mixture.

In another aspect, the present application provides a kit for analyzing a sample including the combination.

In some embodiments, the kit includes at least one of a nucleic acid amplifying agent, a linking agent, an immobilizing agent, a permeating agent, and a lysing agent.

In some embodiments, the kit contains an instruction for use.

In some embodiments, the instruction for use includes the following steps:
contacting at least one target molecule in a sample with at least one probe, the probe including a target molecule binding domain and a probe identity tag;
attaching the probe to a sample-specific barcode to obtain a barcoded probe; and
determining the composition of the barcoded probe, and determining the presence and/or content of the target molecule in the sample from the composition of the barcoded probe.

Other aspects and advantages of the present application can be readily perceived by those skilled in the art from the following detailed description. In the following detailed description, only exemplary embodiments of the present application are shown and described. As will be recognized by those skilled in the art, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

### Brief Description of the Drawings

The specific features of the invention involved in the present application are as shown in the appended claims. The characteristics and advantages of the invention involved in the present application can be better understood by referring to the exemplary embodiments described in detail below and the accompanying drawings. A brief description of the drawings is as below:
Fig. 1 shows a schematic diagram of the structure of an exemplary split probe as described in the present application.
Fig. 2 shows a schematic diagram of the structure of an exemplary padlock probe as described in the present application.
Fig. 3 shows a schematic diagram of obtaining a snail probe by an exemplary PCR method as described in the present application.
Fig. 4 shows a schematic diagram of obtaining a snail probe by an exemplary linking method as described in the present application.
Fig. 5 shows the number of cells predicted by the analytical method as described in the present application. The abscissa shows that the number of cell barcodes sorted by number of reads [descending], and the ordinate shows the cumulative function of reads.
Fig. 6 shows the detection results of the number of genes, the number of mRNA molecules and the mitochondrial reads by the analysis method as described in the present application.
Fig. 7 shows the detection results of an exemplary single cell gene expression matrix by the analysis method as described in the present application.

### Detailed Description of the Embodiments

The implementation of the present invention are described below with reference to specific embodiments, and other advantages and effects of the present invention will be readily known to those familiar with the art from the contents disclosed in the present specification.

### Definition of terms

In the present application, the term "sequencing" generally refers to a technique for obtaining sequence information of nucleic acid molecules. For example, analyzing the base sequence of a specific DNA fragment (e.g., the arrangement of adenine (A), thymine (T), cytosine (C) and guanine (G), etc.). Sequencing methods can include Sanger's dideoxy chain termination method, pyrosequencing method, and "synthetic parallel sequencing" or "sequencing by ligation" platforms used by Illumina, Life Technologies and Roche for new production sequencing, etc., and sequencers made by MGI/Complete Genomics. Generally, it can also include nanopore sequencing methods, such as the methods developed by Oxford Nanopore Technologies, PacBio's third-generation sequencer, or methods based on electronic detection, such as Ion Torrent technology introduced by Life Technologies, etc.

In the present application, the term "solid support" generally refers to any materials suitable for, or that can be modified to be suitable for, attachment of oligonucleotide tags, sample-specific barcodes, primers, etc. as described herein. For example, a solid support includes an array of pores or depressions in a surface that can be fabricated using a variety of techniques, such as photolithography, stamping, molding, and microetching. The composition and geometry of the solid support may vary depending on its use, for example, the solid support may be a planar structure (e.g., glass slides, chips, microchips and/or arrays, etc.). For example, the solid support or its surface may also be non-planar, such as the inner or outer surface of a tube or container. For example, the solid support may also include microspheres or beads.

In the present application, the terms "beads" or "microspheres" or "particles" generally refer to small discrete particles. Suitable bead compositions include, but are not limited to, plastics, ceramics, glass, polystyrene, methylstyrene, acrylic polymers, paramagnetic materials, thorium oxide sol, carbon graphite, titanium dioxide, latex or cross-linked dextran (such as agarose), cellulose, nylon, cross-linked micelles and Teflon, and any other materials outlined herein for solid support may all be used with reference to the Microsphere Detection Guide of Bangs Laboratories, Fishers Ind.. In some embodiments, the microspheres may be magnetic microspheres or beads.

In the present application, the term "unique molecular identification region" may also be referred to as "molecular barcode", "molecular marker", "Unique identifier (UID)", "Unique molecular identifier (UMI)", etc., and generally refers to encoding a unique sequence on each probe linkage. It can typically be designed as a completely random nucleotide chain (e.g., NNNNNNN), a partially degenerate nucleotide chain (e.g., NNNRNYN), or a designated nucleotide chain (e.g., when template molecules are limited). The design, incorporation, and application of UMI may be carried out in a manner known in the art, for example as disclosed in WO 2012/142213 to Islam et, al. (Nat. Methods) (2014) 11:163-166, and (Nat. Methods) (2012) 9: 72-74 to Kivioja, T. et, al., which are incorporated herein by reference in their entirety.

In the present application, the term "amplification primer recognition region" generally refers to a nucleotide sequence capable of complementary hybridization with a primer sequence for amplifying the target nucleic acid. Its binding with the primer can initiate nucleotide extension, ligation and/or synthesis, such as copy number increase (i.e., amplification) of the target nucleic acid under polymerase chain reaction, and in some embodiments, it also includes amplification of oligonucleotide tags, molecular unique identifiers, and other sequences.

In the present application, the term "discrete compartment" generally refers to independent spatial units containing the substance of interest to be analyzed, such as microdroplets or pores. For example, the probe is co-dispensed with a solid support attached with a PCR tag to form a microdroplet; for example, the probe is co-dispensed with a solid support attached with an oligonucleotide tag to form a microdroplet; for example, the probe is co-dispensed with a solid support attached with a PCR tag into the pores of a 96-well plate; for example, the probe is co-dispensed with a solid support attached with an oligonucleotide tag into the pores of a 96-well plate. In some embodiments, the discrete compartments may also include other substances dispensed according to different needs, such as dyes, emulsifiers, surfactants, stabilizers, polymers, aptamers, reducing agents, initiators, biotin markers, fluorophore, buffers, acidic solutions, alkaline solutions, light-sensitive enzymes, pH-sensitive enzymes, aqueous buffers, detergents, ionic detergents, nonionic detergents, etc.

In the present application, the term "releasably attachment" generally means that the attachment between an oligonucleotide tag or PCR tag and a solid support is releasable, cleavable, or reversible, or breakable, eliminable. For example, the ligation of an oligonucleotide tag or PCR tag to a solid support contains unstable bonds, such as chemical, thermal, or light sensitive bonds, e.g., disulfide bonds, UV sensitive bonds, etc., and these unstable bonds can be destroyed by corresponding treatment to achieve releasable attachment. For example, the ligation of an oligonucleotide tag or PCR tag to a solid support contains a specific base that can be recognized by a nuclease, such as a dU, which can be cleaved through the action of a UNG enzyme. For example, the ligation of an oligonucleotide tag or PCR tag to a solid support contains an endonuclease recognition sequence which can be cleaved through the action of a nuclease. For example, the solid support is degradable, and the oligonucleotide tag can be released by degradation of the solid support when degradation conditions are applied, to achieve the releasable attachment.

In the present application, the term "linker" generally refers to a nucleotide sequence linking various functional sequences together, and may also include a molecular sequence (nucleic acid, polypeptide or other chemically linked structures, etc.) that links an oligonucleotide tag or PCR tag to a solid support, wherein the functional sequence may include a cell barcode segment, a sample-specific barcode, an amplification primer recognition region, a sequencing primer recognition region, a unique molecular identifier, etc. In some embodiments, the nucleotide may be an immobilized nucleotide sequence, and in some embodiments, the linker may also include chemical modifications.

In the present application, the term "sample-specific barcode" generally refers to a nucleotide sequence or a derived or modified form thereof that can identify the source of the target molecule.

In the present application, the term "cell barcode" generally refers to a nucleotide sequence that can be used to recognize the source of a target molecule. The source may be, for example, from the same cell or different cells. When the target molecule is derived from multiple sources, different cell barcodes can be used to label the target molecules in each source, so that the sources of the samples can be recognized. Barcodes (also commonly referred to as indexes, tags, etc.) are well known to those skilled in the art, and any suitable barcodes or barcode groups may be used, such as the cell barcodes as described in the publication of US2013/0274117.

In the present application, the term "cell barcode segment" generally refers to the barcode nucleotide units that make up the cell barcode, N of which may form a cell barcode segment by the action of PCR or DNA ligase. N may be greater than or equal to 1 such that the cell barcode formed is sufficient to identify the cell source of each target molecule derived from a plurality of sources.

In the present application, the term "oligonucleotide adapter" generally refers to a nucleotide sequence in a probe that can be linked to the oligonucleotide tag. The nucleotide sequence may be a partially double-stranded structure, for example, it may have a prominent sequence that hybridizes with an oligonucleotide tag. In some embodiments, the oligonucleotide adapter may also include a transposase (e.g., Tn5 transposase) binding sequence. In some embodiments, the oligonucleotide adapter may also include an amplification primer recognition sequence.

In the present application, the term "barcoded probe" generally refers to a probe to which at least a cell barcode is attached.

In the present application, the terms "hybridized," "hybridizable," or "complementary" generally mean that a nucleic acid (e.g., RNA, DNA) includes a nucleotide sequence capable of specifically binding to another nucleic acid sequence non-covalently (i.e., forming a Watson-Crick base pair and/or a G/U base pair) under *in vitro* and/or *in vivo* conditions of appropriate temperature and solution ionic strength. The Watson-Crick base pairing includes: adenine/adenosine (A) paired with thymidine/thymine (T), A paired with uracil/uridine (U), guanine/guanosine (G) paired with cytosine/cytidine (C). In some embodiments, G may also be paired with a U base for hybridization between two RNA molecules (e.g., dsRNA), or for hybridization between DNA molecules and RNA molecules (e.g., when the bases of a DNA target nucleic acid are paired with a guide RNA, etc.). Hybridization requires that the two nucleic acids contain complementary sequences, but the possible mismatch between bases cannot be ruled out. The conditions suitable for hybridization between two nucleic acids depend on the length and degree of complementarity of the nucleic acids, which are well known in the art. The greater the degree of complementarity between two nucleotide sequences, the greater the value of the melting temperature (Tm) of the hybrids of nucleic acids having these complementary sequences.

In the present application, the term "reads" generally refers to a sequencing sequence obtained by a reaction in nucleotide sequencing. Reads can be a short sequencing fragment, which is the base sequence data obtained from a single sequencing by a sequencer. The length of reads can be different with different sequencing instruments.

### Detailed description of the invention

In one aspect, the present application provides a method for analyzing a target molecule in a sample, the method including:
contacting at least one target molecule in a sample with at least one probe, the probe including a target molecule binding domain;
attaching the probe to a sample-specific barcode to obtain a barcoded probe; and
determining the composition of the barcoded probe, and determining the presence and/or content of the target molecule in the sample from the composition of the barcoded probe.

In another aspect, the present application provides a method for amplifying a target molecule in a sample, the method including:
contacting at least one target molecule in a sample with at least one probe, the probe including a target molecule binding domain;
attaching the probe to a sample-specific barcode to obtain a barcoded probe;
amplifying the barcoded probe; and
determining the composition of the barcoded probe, and determining the presence and/or content of the target molecule in the sample from the composition of the barcoded probe.

In another aspect, the present application provides a method for sequencing a target molecule in a sample, the method including:
contacting at least one target molecule in a sample with at least one probe, the probe including a target molecule binding domain;
attaching the probe to a sample-specific barcode to obtain a barcoded probe; and
sequencing the barcoded probe, and determining the sequencing result of the barcoded probe and determining the presence and/or content of the target molecule in the sample from the sequencing result of the barcoded probe.

### Sample

In the present application the sample may be derived from almost any organism and may include multicellular organisms such as plants, fungi and animals. For example, the sample may be derived from an animal (e.g., a mammal). For example, the sample may be derived from human origin. For example, the sample may be a biopsy tumor or a part thereof. For example, the sample may be a clinically relevant tissue sample. For example, the tumor may include a solid tumor. For example, the tumor may include a hematological tumor.

In the present application, the sample may contain any number of substances, including ( but not limited to ) : cells of almost any organism ( including both primary cells and cultured cell lines ), cell lysates or extracts ( including ( but not limited to ) RNA extracts, purified mRNA ), tissue and tissue extracts ( including ( but not limited to ) RNA extracts, purified mRNA ), body fluids ( including ( but not limited to ) blood, urine, serum, lymph, bile, cerebrospinal fluid, interstitial fluid, aqueous or glassy fluid, colostrum, sputum, amniotic fluid, saliva, anal and vaginal secretions, sweat and semen, exudate, exudate (e.g., from abscesses or any other site of infection or inflammation ) or from joints (e.g., normal joints or joints affected by diseases such as rheumatoid arthritis, osteoarthritis, gout or suppurative arthritis ) ; the study samples included extracellular fluid, extracellular supernatant from cell culture, inclusion bodies in bacteria, cell compartments, periplasm, and mitochondrial compartments.

In the present application, the sample may include at least one cell. For example, the samples may include 1 or more, 2 or more, 3 or more, 4 or more, 5 or more, or 6 or more. For example, the sample may be a single cell.

### Probes and target molecules

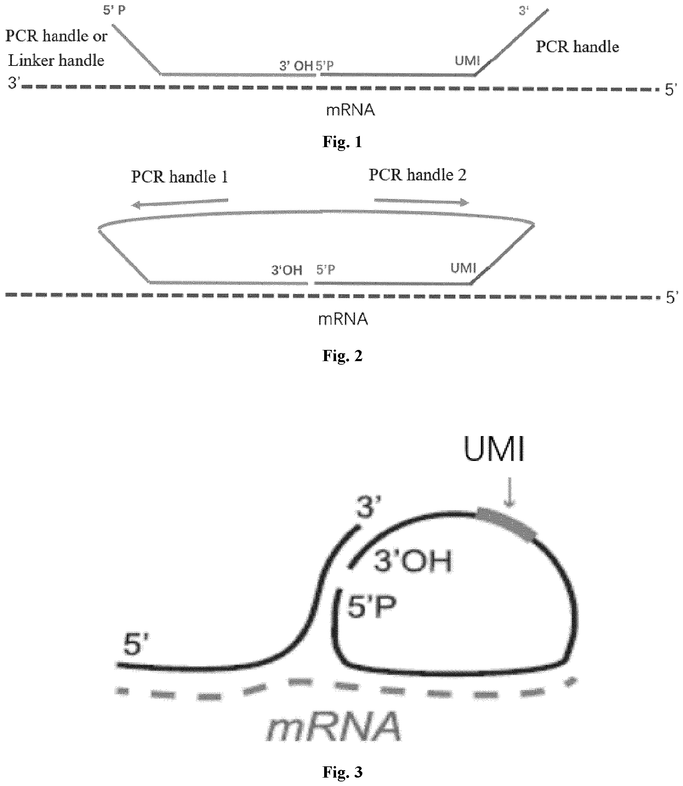

In the present application, probes can be designed by commonly used methods. For example, the probe can be designed by Oligo 6. For example, probes can be designed by BLAST. For example, probes can be designed by Beacon Designer 7.0. For example, a probe can be designed by Primer Express 3.0. For example, a probe can be designed by Primer Premier 5. For example, a probe can be designed by Primer Designer-M. In the present application, the probe can be designed by Primer 3. Exemplary probe configurations are shown in Figs. 1-4.

In the present application, the probe may include a target molecule binding domain. For example, the probe may include a probe identity tag. For example, the probe identity tag generally refers to a unique nucleic acid sequence attached to each of the plurality of probes. For example, when incorporated into a probe, a probe identity tag may be used for probes sequenced by direct counting after amplification. For example, the probe identity tag is directly or indirectly linked to the target molecule binding domain.

In the present application, the probe identification tag may include a unique molecular identification region. For example, the unique molecular identification region may include a single-stranded nucleic acid. For example, a single-stranded nucleic acid in the unique molecular identification region may include 1 or more nucleotides, 2 or more nucleotides, 3 or more nucleotides or 4 or more nucleotides, 5 or more nucleotides, 6 or more nucleotides, 7 or more nucleotides or 8 or more nucleotides, 9 or more nucleotides, 12 or more nucleotides, 15 or more nucleotides or 20 or more nucleotides. For example, a single-stranded nucleic acid in the unique molecular identification region may contain 8 nucleotides.

In the present application, the target molecule may include one or more selected from the group consisting of proteins, nucleic acid molecules, metabolites and/or lipids. For example, the target molecules may include DNA molecules and/or RNA molecules. For example, the DNA molecule may be cDNA. For example, the RNA molecule may include cRNA, mRNA, miRNA, IncRNA and/or eRNA. For example, the RNA may be mRNA. For example, the lipids may include fats, cporesterol, cporesterol esters, phospholipids and/or glycolipids.

In the present application, the target molecule may include mRNA, and the target molecule binding domain in the probe may include a single-stranded nucleic acid. For example, the target molecule hybridizes with the target molecule binding domain. With respect to the hybridization the conditions suitable for hybridization between two nucleic acids depend on the length and degree of complementarity of the nucleic acids which are well known in the art. The greater the degree of complementarity between two nucleotide sequences, the greater the value of the melting temperature (Tm) of the hybrids of nucleic acids having these complementary sequences. For example, the single-stranded nucleic acid may include at least 10 nucleotides. For example, the single-stranded nucleic acid may include 10 nucleotides or more, 12 nucleotides or more, 14 nucleotides or more, 16 nucleotides or more, 18 nucleotides or more, 20 nucleotides or more, 22 nucleotides or more, 24 nucleotides or more, 26 nucleotides or more, 28 nucleotides or more, 30 nucleotides or more, 32 nucleotides or more, 34 nucleotides or more, 36 nucleotides or more, 38 nucleotides or more, 40 nucleotides or more, 42 nucleotides or more, 44 nucleotides or more, 46 nucleotides or more, 48 nucleotides or more, 50 nucleotides or more, 52 nucleotides or more, 54 nucleotides or more, 56 nucleotides or more, 58 nucleotides or more, or 60 nucleotides or more. For example, the hybridization does not exclude possible mismatches between bases. For example, the target molecule binding domain in the probe is at least 50% complementary to the sequence of the mRNA. For example, it can be 50% or more, 55% or more, 60% or more, 65% or more, 70% or more, 75% or more, 80% or more, 85% or more, 90% or more, 95% or more, 98% or more, 99% or more, 99.5% or more, 99.5% or more. The remaining non-complementary nucleotides may be clustered or dispersed with the complementary nucleotides and need not be adjacent to each other or to the complementary nucleotides. For example, polynucleotides may hybridize on one or more segments such that intermediate or adjacent segments are not involved in hybridization events (e.g., hairpin formation, "bumps", etc.).

In the present application, the composition of the target molecule may be known. For example, the target molecule may be mRNA, and the nucleic acid sequence of the mRNA may be known.

For example, the target molecule may include a protein. For example, the target molecule may be a protein, and the target molecule binding domain may include an antibody or antigen binding fragment capable of specifically binding the target molecule. For example, the antigen binding fragment may include Fab, Fab', F (ab) 2, Fv fragment, F (ab') 2, scFv, di-scFv, VHH and/or dAb. In the present application, the identity of the protein may be known. For example, the amino acid sequence of the protein may be known.

In the present application, at least one target molecule in the sample can be contacted with two or more probes. For example, at least one target molecule in the sample can be contacted with the first probe, and the target molecule can be contacted with a second probe different from the first probe. For example, the 3' end of the first probe may be linked directly or indirectly to the 5' end of the second probe. For example, the 5' end of the first probe may be linked directly or indirectly to the 3' end of the second probe. For example, the 3' end of the first probe may be linked to the 5' end of the second probe by a ligase. For example, the 5' end of the first probe may be linked to the 3' end of the second probe by a ligase. For example, the first probe may form a single-stranded cyclic molecule with the second probe. For example, the nucleic acid sequence at the 3' end of the first probe may be complementary to the nucleic acid sequence at the 3' end of the second probe, the nucleic acid sequence of the first probe may be complementary to the nucleic acid sequence at the 5' end of the second probe, and the 5' end of the second probe may be directly or indirectly linked to the 3' end of the second probe. For example, the 5' end of the second probe may be linked to the 3' end of the second probe by a ligase.

For example, the ligase may include DNA ligases such as *Escherichia coli* DNA ligase, T4 DNA ligase, T7 DNA ligase, mammalian ligase (e.g., DNA ligase I, DNA ligase III, DNA ligase IV), thermostable ligase, etc. T4 DNA ligase can link segments containing DNA, oligonucleotides, RNA and RNA-DNA hybrids. The ligation reaction may not include DNA ligase, but a substitute, such as topoisomerase, is used. High concentration of DNA ligase and PEG can realize rapid ligation. In order to select a favorable temperature for the ligation reaction, the optimal temperature of the DNA ligase (e.g., 37°C) and the melting temperature of the DNA to be ligated can be considered. The target nucleic acid and the barcoded solid support may be suspended in a suitable buffer to minimize ionic interactions that may affect the ligation. For example, the ligase may include a T4 ligase.

In the present application, two or more probes can be designed for known genes. For example, the probe may include an amplification primer recognition region. For example, the probe may include an oligonucleotide tag. For example, in the probe, the amplification primer binding region may be directly or indirectly linked to the probe identity tag. For example, at least two of the two or more probes can bind to different regions of the same target molecule in the sample.

### PCR tag

In the present application, the PCR tag may include a sample-specific barcode and a PCR handle sequence at the 3' end of the sample-specific barcode, the PCR handle sequence being same to the sequence at the 5' end of the probe.

In the present application, the PCR tag may include a sample-specific barcode and a PCR handle sequence at the 3' end of the sample-specific barcode, the PCR handle sequence being same to the sequence at the 3' end of the probe.

In the present application, a solid support to which at least one PCR tag is attached may be provided prior to contacting a target molecule in a sample with the probe and prior to attaching the probe to a sample-specific barcode, wherein the sequence in the PCR handle in each of the PCR tags is same to the sequence at the 5' end of the probe, and free primers may be provided which are complementary to the sequence at the 3' end of the probe. In the present application, the method may further include co-dispensing the probe, the solid support to which at least one PCR tag is attached, and the free primer into discrete compartments, and the probe may be attached with a sample-specific barcode.

In the present application, a solid support to which at least one PCR tag is attached may be provided before to contacting a target molecule in a sample with the probe and before to attaching the probe to a sample-specific barcode, wherein the sequence in the PCR handle in each of the PCR tags is same to the sequence at the 3' end of the probe, and free primers may be provided which are complementary to the sequence at the 5' end of the probe. In the present application, the method may further include co-dispensing the probe, the solid support to which at least one PCR tag is attached, and the free primer into discrete compartments, and the probe may be attached with a sample-specific barcode.

In the present application, the PCR tag is releasably attached to the solid support. For example, the PCR tags attached to the same solid support can be the same or different. In the present application, the PCR handle in the PCR tag may include 1 or more nucleotide sequences, for example, the PCR handle may have 2 or more, 3 or more, 4 or more, 5 or more, 6 or more, 7 or more, 8 or more, 9 or more, 10 or more, 11 or more, 12 or more, 13 or more, 14 or more, or 15 or more sequences.

For example, the PCR tags attached to the solid support and containing different kinds of the PCR handles may be 1 or more, for example, 50 or more, 100 or more, 500 or more, 1000 or more, 1500 or more, 2000 or more, 3000 or more, 5000 or more, 8000 or more, 10000 or more, 12,000 or more, 15,000 or more, 18,000 or more, 20,000 or more, 22,000 or more, 25,000 or more, 28,000 or more, 30,000 or more, 35,000 or more, 40,000 or more, 45,000 or more, 50,000 or more.

For example, the number of PCR tags attached to the same solid support and containing the same kind of PCR handle may be set to different proportions as desired.

For example, a PCR tag attached to the same solid support may include 1 or more PCR tags, for example, 50 or more, 100 or more, 500 or more, 1,000 or more, 1,500 or more, 2,000 or more, 3,000 or more, 5,000 or more, 8,000 or more, 10,000 or more, 12,000 or more, 15,000 or more, 18,000 or more, 20,000 or more, 22,000 or more, 22,000 or more, 25,000 or more, 28,000 or more, 30,000 or more, 35,000 or more, 40,000 or more, 45,000 or more, 50,000 or more, 55,000 or more, 60,000 or more, 65,000 or more, 70,000 or more, 75,000 or more, 80,000 or more, 85,000 or more, 90,000 or more, 95,000 or more, 100,000 or more, 110,000 or more, 120,000 or more, the sample-specific barcodes of these PCR tags may be the same.

For example, the cell barcodes contained in the PCR tag set attached to different solid supports can be different from each other, and the PCR tag set can be a combination of all PCR tags attached to the same solid support.

In the present application, the sample-specific barcode may include a cell barcode. For example, the cell barcode includes at least two cell barcode segments. For example, the cell barcode segment is 4 or more nucleotides (nt), for example, 5 or more, for example, 10 or more, 12 or more, 15 or more, 18 or more, 20 or more, 21 or more, 22 or more, 23 or more, 24 or more, 25 or more, 26 or more, 27 or more, 28 or more, 29 or more, 30 or more, 31 or more, 32 or more, 33 or more, 34 or more, or 35 or more.

For example, the cell barcode includes at least 2 cell barcode segments, at least 3 cell barcode segments, at least 4 cell barcode segments, at least 5 cell barcode segments, at least 6 cell barcode segments, at least 7 cell barcode segments, and at least 8 cell barcode segments, and the cell barcode segments are encoded as cell barcode segment 1, cell barcode segment 2, cell barcode segment 3, cell barcode segment 4, cell barcode segment 5, and cell barcode segment n according to the sequence from 5' end to 3' end in the PCR tag. For example, the at least 2 cell barcode segments may form the cell barcode by PCR or DNA ligase.

For example, the cell barcode may be produced by:
1) dividing at least one of the solid supports into at least 2 primary aliquots, for example, at least 8 aliquots, at least 16 aliquots, at least 24 aliquots, at least 32 aliquots, at least 40 aliquots, at least 48 aliquots, at least 56 aliquots, at least 64 aliquots, at least 72 aliquots, at least 80 aliquots, at least 88 aliquots, at least 96 aliquots;
2) providing each of the primary aliquots with at least one cell barcode segment 1, for example, at least 1,000 cell barcode segments 1, for example, at least 10,000 cell barcode segments 1, for example, at least 100,000 cell barcode segments 1, for example, at least 1,000,000 cell barcode segments 1, for example, at least 10,000,000 cell barcode segments 1, the sequence and/or length of cell barcode segments 1 in each aliquot and in any other aliquot are different from each other;
3) linking at least one solid support in each of the primary aliquots directly or indirectly to a cell barcode segment 1, each solid support being linked to at least one cell barcode segment 1;
4) combining the at least two of the primary aliquots, dividing the combined primary aliquots into at least two secondary aliquots, for example, at least 8 aliquots, at least 16 aliquots, at least 24 aliquots, at least 32 aliquots, at least 40 aliquots, at least 48 aliquots, at least 56 aliquots, at least 64 aliquots, at least 72 aliquots, at least 80 aliquots, at least 88 aliquots, at least 96 aliquots;
5) providing each of the secondary aliquots with at least one cell barcode segment 2 or a complementary sequence thereof, for example, at least 1,000 cell barcode segments 2 or a complementary sequence thereof, for example, at least 10,000 cell barcode segments 2 or a complementary sequence thereof, for example, at least 100,000 cell barcode segments 2 or a complementary sequence thereof, for example, at least 10,000,000 cell barcode segments 2 or a complementary sequence thereof, the sequence and/or length of the cell barcode segment 2 or a complementary sequence thereof in each aliquot is different from that of any other aliquot;
6) linking at least one cell barcode segment 1 linked to a solid support in each of the secondary aliquots directly or indirectly to a cell barcode segment 2.

For example, steps 4)-6) may be repeated for n times, and n may be 1, 2, 3, 4, 5, 6, 7, 8, 9, 10 or more, to connect cell barcode segment 3, cell barcode segment 4, cell barcode segment 5 ...... cell barcode segment n to generate a cell barcode that is sufficient to target the unique sequence of each cell, so that a target molecule in the first cell can have a first cell barcode with a unique sequence, a target molecule in the second cell can have a second cell barcode with a unique sequence, a target molecule in the second cell can have a second cell barcode with a unique sequence, and so on.

For example, the PCR tag also contains the linker sequence 1, and the 5' end of the cell barcode segment 1 may be linked to the solid support through the linker sequence 1. The linker sequence 1 may include acrydite modification, light cutting modification, S-S modification, dU base modification and other sequences, which can be interrupted by various methods to release the PCR tags.

For example, the PCR tag may also contain other functional sequences, such as full or partial functional sequences (e.g., primer sequence (e.g., universal primer sequence, targeting primer sequence, random primer sequence) recognition region, primer annealing sequence, attachment sequence, sequencing primer recognition region, amplification primer recognition region (e.g., universal amplification primer recognition region), etc. for subsequent processing.

For example, the subsequent processing includes amplification. For example, the amplification may include PCR amplification (e.g., Taq DNA polymerase amplification, Super Taq DNA polymerase amplification, LA Taq DNA polymerase amplification, Pfu DNA polymerase amplification, Phusion DNA polymerase amplification, KOD DNA polymerase amplification, etc.), isothermal amplification (e.g., may include loop mediated isothermal amplification (LAMP), helicase dependent amplification (HDA), recombinase polymerase amplification (RPA), strand displacement amplification (SDA), nucleic acid sequence based amplification (NASBA), transcription mediated amplification (TMA), etc.), T7 promoter linear amplification, degenerate oligonucleotide primer PCR amplification (DOP-PCR), multiple displacement amplification (MDA), multiple annealing and looping-based amplification cycles (MALBAC), etc.

For example, the cell barcode may also not contain linkers, and the cell barcode may be a separate nucleic acid sequence synthesized by other methods.

For example, the universal primer sequence may include P5 or other suitable primers. The universal primer (e.g., P5) may also be compatible with the sequencing device such as being able to attach to a flow cell within the sequencing devices. For example, such universal primer sequences may provide complementary sequences of oligonucleotides constrained to the surface of a flow cell in a sequencing device to enable a barcoded probe to be immobilized on the surface for sequencing.

For example, amplification primer sequences, primer sequences used to perform amplification or replication processes (e.g., extending primers along target nucleic acid sequences) to produce amplified barcoded probe sequences.

For example, sequencing primer sequences, the resulting amplification target sequence will contain such primers and be easily transferred into the sequencing system. For example, when the amplified target is sequenced using the Illumina sequencing system, the sequencing primer sequence may include a Read1 primer sequence, a Read2 primer sequence.

For example, the PCR tag may contain a T7 promoter sequence. For example, a ligation reaction is used to link barcode segments of cells to form PCR tags. The linkage may include bonding two nucleic acid segments together by catalyzing the formation of phosphodiester bonds, such as cell barcode segment 1 and the functional sequence described above, such as linker sequence 2 and cell barcode segment 2, linker sequence 3 and cell barcode segment 3, linker sequence 4 and cell barcode segment 4, linker sequence 5 and cell barcode segment 5, linker sequence 6 and cell barcode segment 6, and so on. The ligation reaction may include DNA ligases such as *Escherichia coli* DNA ligase, T4 DNA ligase, mammalian ligase (e.g., DNA ligase I, DNA ligase III, DNA ligase IV), thermostable ligase, etc. T4 DNA ligase can link segments containing DNA, oligonucleotides, RNA and RNA-DNA hybrids. The ligation reaction may not include DNA ligase, but a substitute, such as topoisomerase, is used. High concentration of DNA ligase and PEG can realize rapid ligation. In order to select a favorable temperature for the ligation reaction, the optimal temperature of the DNA ligase (e.g., 37°C) and the melting temperature of the DNA to be ligated can be considered. The sample and the barcoded solid support may be suspended in a buffer to minimize ionic interaction that may affect the ligation.

For example, each cell barcode segment is ligated by polymerase chain reaction (PCR) to form a PCR tag. For example, the polymerase chain reaction may be performed by any one or more of the following polymerases: Taq DNA polymerase, Super Taq DNA polymerase, LA Taq DNA polymerase, UlltraPF DNA polymerase, Tth DNA polymerase, Pfu DNA polymerase, VentR DNA polymerase, Phusion DNA polymerase, KOD DNA polymerase, Iproof DNA polymerase. For example, the polymerase chain reaction may also include a buffer for keeping the polymerase active, a metal ion; For example, dNTP and or modified derivatives thereof may also be included in the polymerase chain reaction.

For example, under the condition of polymerase chain reaction (PCR) to produce PCR tags, each round provides a complementary sequence of the cell barcode segment, the complementary sequence being a single-stranded structure, wherein the 5'terminal linker sequence can be at least partially complementarily paired with the 3'terminal linker sequence of the cell barcode segment linked in the previous round to form a double-stranded structure.

### Oligonucleotide tag

In the present application, the probe may also include an oligonucleotide adapter. For example, the oligonucleotide adapter may be located at the 5' end of the probe. For example, the oligonucleotide adapter may also be located at the 3' end of the probe.

In the present application, the oligonucleotide tag may include a single-stranded nucleic acid, and the oligonucleotide tag may include a sample-specific barcode and a hybridization sequence at the 3' end of the sample-specific barcode.

In the present application, the oligonucleotide tag may contain a first strand and a second strand. The first strand contains a sample-specific barcode and a hybridization sequence at the 3' end of the sample-specific barcode. The second strand contains the first portion complementary to the hybridization sequence of the first strand and the second portion complementary to the sequence of the oligonucleotide adapter in the probe, and the first and second strands forming a partially double-stranded structure.

With respect to the hybridization sequence the conditions suitable for hybridization between two nucleic acids depend on the length and degree of complementarity of the nucleic acids which are well known in the art. The greater the degree of complementarity between two nucleotide sequences, the greater the value of the melting temperature (Tm) of the hybrids of nucleic acids having these complementary sequences.

For example, the first portion of the second strand in the oligonucleotide tag or the second portion of the second strand in the oligonucleotide tag is sufficiently long to form a double-stranded structure with its complementary sequence (e.g., a hybridization sequence in the first strand in the oligonucleotide tag at the 3' end of the sample-specific barcode, e.g. the oligonucleotide adapter sequence or part thereof in the probe).

For example, the length of the first portion of the second strand in the oligonucleotide tag or the second portion of the second strand in the oligonucleotide tag may be 1 nucleotide or more, 2 nucleotides or more, 3 nucleotides or more, 5 nucleotides or more, 8 nucleotides or more, 10 nucleotides or more, 12 nucleotides or more, 15 nucleotides or more, 20 nucleotides or more, 22 nucleotides or more, 25 nucleotides or more, or 30 nucleotides or more.

For example, the length of the sequence of the first portion of the second strand in the oligonucleotide tag and the second portion of the second strand in the oligonucleotide tag may be the same or different.

For example, the double-stranded structure does not exclude possible mismatches between bases. For example, the sequence of the first portion of the second strand in the oligonucleotide tag or the second portion of the second strand in the oligonucleotide tag need not be 100% complementary to the sequence of its hybridization sequence. For example, it can be 60% or more, 65% or more, 70% or more, 75% or more, 80% or more, 85% or more, 90% or more, 95% or more, 98% or more, 99% or more, 99.5% or more complementary. The remaining non-complementary nucleotides may be clustered or dispersed with the complementary nucleotides and need not be adjacent to each other or to the complementary nucleotides. For example, polynucleotides may hybridize on one or more segments such that intermediate or adjacent segments are not involved in hybridization events (e.g., hairpin formation, "bumps", etc.).

For example, the second portion of the oligonucleotide tag attached to the same solid support may be same.

For example, the second portion of the oligonucleotide tag attached to the same solid support may be different. For example, the second portion of each oligonucleotide tag attached to the same solid support may include 1 or more nucleotide sequences, for example, the second portion may have 2 or more sequences, for example, 3 or more, for example, 4 or more, for example, 5 or more, for example, 6 or more, for example, 7 or more, for example, 8 or more, for example, 9 or more, for example, 10 or more, for example, 11 or more, for example, 12 or more, for example, 13 or more, for example, 14 or more, for example, 15 or more, so that the oligonucleotide tag attached to the same solid support can be linked to a corresponding one or more of the probes.

For example, the number of the oligonucleotide tags attached to the same solid support and containing the same second portion may be 1 or more, for example, 50 or more, 100 or more, 500 or more, 1,000 or more, 1,500 or more, 2,000 or more, 3,000 or more, 5,000 or more, 8,000 or more, 10,000 or more, 12,000 or more, 15,000 or more, 18,000 or more, 20,000 or more, 22,000 or more, 25,000 or more, 28,000 or more, 30,000 or more, 35,000 or more, 40,000 or more, 45,000 or more, 50,000 or more.

For example, the number of oligonucleotide tags containing different second moieties attached to the same solid support may be set in different proportions as desired to attach to the corresponding probe.

For example, the sample-specific barcode includes a cell barcode, and each oligonucleotide tag attached to the same solid support includes the same cell barcode.

For example, an oligonucleotide tag attached to the same solid support may include one or more oligonucleotide tags, For example, 50 or more, 100 or more, 500 or more, 1,000 or more, 1,500 or more, 2,000 or more, 3,000 or more, 5,000 or more, 8,000 or more, 10,000 or more, 12,000 or more, 15,000 or more, 18,000 or more, 20,000 or more, 22,000 or more, 25,000 or more, 28,000 or more, 30,000 or more, 35,000 or more, 40,000 or more, 45,000 or more, 50,000 or more, 55,000 or more, 60,000 or more, 65,000 or more, 70,000 or more, 75,000 or more, 80,000 or more, 85,000 One or more, 90,000 or more, 95,000 or more, 100,000 or more, 110,000 or more, 120,000 or more, the cell barcodes of these oligonucleotide tags are the same, and the sequence of the second portion of the second strand thereof may be one or more, for example, the sequence of the second portion is 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more.

For example, the oligonucleotide tag groups attached to different solid supports contain different cell barcodes. The oligonucleotide tag group can be a combination of all oligonucleotide tags attached to the same solid support.

For example, the cell barcode includes at least two cell barcode segments. For example, the cell barcode segment is 4 or more nucleotides (nt), for example, 5 or more, for example, 10 or more, 12 or more, 15 or more, 18 or more, 20 or more, 21 or more, 22 or more, 23 or more, 24 or more, 25 or more, 26 or more, 27 or more, 28 or more, 29 or more, 30 or more, 31 or more, 32 or more, 33 or more, 34 or more, or 35 or more.

For example, the cell barcode includes at least 2 cell barcode segments, at least 3 cell barcode segments, at least 4 cell barcode segments, at least 5 cell barcode segments, at least 6 cell barcode segments, at least 7 cell barcode segments, at least 8 cell barcode segments, and the cell barcode segments are encoded as cell barcode segment 1, cell barcode segment 2, cell barcode segment 3, cell barcode segment 4, cell barcode segment 5... cell barcode segment n according to the sequence from 5' end to 3' end in the oligonucleotide tag. For example, at least two cell barcode segments may form a cell barcode by PCR or DNA ligase.

For example, the cell barcode may be produced by:
1) dividing at least one of the solid supports into at least 2 primary aliquots, for example, at least 8 aliquots, at least 16 aliquots, at least 24 aliquots, at least 32 aliquots, at least 40 aliquots, at least 48 aliquots, at least 56 aliquots, at least 64 aliquots, at least 72 aliquots, at least 80 aliquots, at least 88 aliquots, at least 96 aliquots;
2) providing each of the primary aliquots with at least one cell barcode segment 1, for example, at least 1,000 cell barcode segments 1, for example, at least 10,000 cell barcode segments 1, for example, at least 100,000 cell barcode segments 1, for example, at least 1,000,000 cell barcode segments 1, for example, at least 10,000,000 cell barcode segments 1, the sequence and/or length of cell barcode segments 1 in each aliquot and in any other aliquot are different from each other;
3) linking at least one solid support in each of the primary aliquots directly or indirectly to a cell barcode segment 1, each solid support being linked to at least one cell barcode segment 1;
4) combining at least two primary aliquots, dividing the combined primary aliquots into at least two secondary aliquots, for example, at least 8 aliquots, at least 16 aliquots, at least 24 aliquots, at least 32 aliquots, at least 40 aliquots, at least 48 aliquots, at least 56 aliquots, at least 64 aliquots, at least 72 aliquots, at least 80 aliquots, at least 88 aliquots, at least 96 aliquots;
5) providing each of the secondary aliquots with at least one cell barcode segment 2 or a complementary sequence thereof, for example, at least 1,000 cell barcode segments 2 or a complementary sequence thereof, for example, at least 10,000 cell barcode segments 2 or a complementary sequence thereof, for example, at least 100,000 cell barcode segments 2 or a complementary sequence thereof, for example, at least 10,000,000 cell barcode segments 2 or a complementary sequence thereof, the sequence and/or length of the cell barcode segment 2 or a complementary sequence thereof in each aliquot is different from that of any other aliquot;
6) linking at least one cell barcode segment 1 linked to a solid support in each of the secondary aliquots directly or indirectly to a cell barcode segment 2.

For example, steps 4)-6) may be repeated for n times, and n may be 1, 2, 3, 4, 5, 6, 7, 8, 9, 10 or more, to connect cell barcode segment 3, cell barcode segment 4, cell barcode segment 5 ...... cell barcode segment n to generate a cell barcode that is sufficient to target the unique sequence of each cell, so that a target molecule in the first cell can have a first cell barcode with a unique sequence, a target molecule in the second cell can have a second cell barcode with a unique sequence, a target molecule in the second cell can have a second cell barcode with a unique sequence, and so on.

For example, the oligonucleotide tag further includes a linker sequence 1 through which the 5' end of the cell barcode segment 1 can be linked to a solid support. The linker sequence 1 may include acrydite modification, photocleavage modification, S-S modification, dU base modification and other sequences, and can be interrupted by various methods to release oligonucleotide tags.

For example, the oligonucleotide tag also includes other functional sequences, such as full or partial functional sequences (e.g., primer sequence (e.g., universal primer sequence, targeting primer sequence, random primer sequence) recognition region, primer annealing sequence, attachment sequence, sequencing primer recognition region, amplification primer recognition region (e.g., universal amplification primer recognition region), etc. for subsequent processing.

For example, the subsequent processing includes amplification. For example, the amplification may include PCR amplification (e.g., Taq DNA polymerase amplification, Super Taq DNA polymerase amplification, LA Taq DNA polymerase amplification, Pfu DNA polymerase amplification, Phusion DNA polymerase amplification, KOD DNA polymerase amplification, etc.), isothermal amplification (e.g., may include loop mediated isothermal amplification (LAMP), helicase dependent amplification (HDA), recombinase polymerase amplification (RPA), chain displacement amplification (SDA), nucleic acid sequence based amplification (NASBA), transcription mediated amplification (TMA), etc.), T7 promoter linear amplification, degenerate oligonucleotide primer PCR amplification (DOP-PCR), multiple displacement amplification (MDA), multiple annealing and looping-based amplification cycles (MALBAC), etc.

For example, the cell barcode may also contain no linker, and the cell barcode may be a separate nucleic acid sequence synthesized by other methods.

For example, the universal primer sequence may include P5 or other suitable primers. The universal primer (e.g., P5) may also be compatible with the sequencing device such as being able to attach to a flow cell within the sequencing device. For example, such universal primer sequences may provide complementary sequences of oligonucleotides that constrain the surface of a flow cell in a sequencing device to enable a barcoded probe to be immobilized to the surface for sequencing.

For example, amplification primer sequences, primer sequences used to perform amplification or replication processes (e.g., extending primers along target nucleic acid sequences) to produce amplified barcoded probe sequences.

For example, amplification primer sequences, the resulting amplification target sequence will contain such primers and be easily transferred into the sequencing system. For example, when the amplified target is sequenced using the Illumina sequencing system, the sequencing primer sequence may include a Read1 primer sequence, a Read2 primer sequence.

For example, the oligonucleotide tag may include a T7 promoter sequence.

For example, any of the above-mentioned multiple functional sequences or a combination thereof may be included between the cell barcode segment 1 and the linker sequence 1. For example, these oligonucleotide tags may include any one or more of the following: P5, Read1 and Read2 sequences, non-cleavable 5' acrydite-P5, cleavable 5' acrydite-SS-P5, R1c, sequencing primers, read primers, universal primers, P5_U, universal read primers, and/or binding sites of any of these primers.

For example, the cell barcode includes at least two cell barcode segments spaced by a linker sequence.

For example, the 3' end of cell barcode segment 1 has a linker sequence 2, the 5' and 3' ends of cell barcode segment 2 have linker sequences 3 and 4 respectively, the 5' and 3' ends of cell barcode segment 3 have linker sequences 5 and 6 respectively, the 5' and 3' ends of cell barcode segment 4 have linker sequences 7 and 8 respectively, and so on, the 5' and 3' ends of cell barcode segment n have linker sequences 2n-1 and 2n respectively. The linker sequence 2 and linker sequence 3 can be at least partially complementarily paired to form a double-stranded structure, the linker sequence 4 and linker sequence 5 can be at least partially complementarily paired to form a double-stranded structure, the linker sequence 6 and linker sequence 7 can be at least partially complementarily paired to form a double-stranded structure, and so on, to initiate the ligation of cell barcode segment 1, cell barcode segment 2, cell barcode segment 3, cell barcode segment 4... cell barcode segment n.

For example, a ligation reaction is used to ligate barcode segments of cells to form oligonucleotide tags. The linkage may include bonding two nucleic acid segments together by catalyzing the formation of phosphodiester bonds, such as cell barcode segment 1 and the functional sequence described above, such as linker sequence 2 and cell barcode segment 2, linker sequence 3 and cell barcode segment 3, linker sequence 4 and cell barcode segment 4, linker sequence 5 and cell barcode segment 5, linker sequence 6 and cell barcode segment 6, and so on. The ligation reaction may include DNA ligases such as *Escherichia coli* DNA ligase, T4 DNA ligase, mammalian ligase (e.g., DNA ligase I, DNA ligase III, DNA ligase IV), thermostable ligase, etc. T4 DNA ligase can link segments containing DNA, oligonucleotides, RNA and RNA-DNA hybrids. The ligation reaction may not include DNA ligase, but a substitute, such as topoisomerase, is used. High concentration of DNA ligase and PEG can realize rapid ligation. In order to select a favorable temperature for the ligation reaction, the optimal temperature of the DNA ligase (e.g., 37°C) and the melting temperature of the DNA to be ligated can be considered. The sample and the barcoded solid support may be suspended in a buffer to minimize ionic interaction that may affect the ligation.

For example, each cell barcode segment is linked by polymerase chain reaction (PCR) to form an oligonucleotide tag. For example, the polymerase chain reaction may be performed by any one or more of the following polymerases: Taq DNA polymerase, Super Taq DNA polymerase, LA Taq DNA polymerase, UlltraPF DNA polymerase, Tth DNA polymerase, Pfu DNA polymerase, VentR DNA polymerase, Phusion DNA polymerase, KOD DNA polymerase, Iproof DNA polymerase. For example, the polymerase chain reaction may also include a buffer for keeping the polymerase active, a metal ion; For example, dNTP and or modified derivatives thereof may also be included in the polymerase chain reaction.

For example, under the condition of polymerase chain reaction (PCR) producing oligonucleotide tags, each round provides a complementary sequence of a cell barcode segment, the complementary sequence being a single-stranded structure, and each of the 5'and 3' ends has a single-stranded linker sequence, wherein the 5' end linker sequence can be at least partially complementarily paired with the 3' end linker sequence of the cell barcode segment linked in the previous round to form a double-stranded structure, and the 3' end linker sequence can be at least partially complementarily paired with the 5' end linker sequence of the cell barcode segment linked in the subsequent round to form a double-stranded structure.

### Barcoded probe

In the present application, after contacting a target molecule in a sample with the probe and before attaching the probe with a sample-specific barcode, providing a solid support attached with at least one PCR tag, where each of the PCR tags includes a sample-specific barcode and a PCR handle sequence at the 3' end of the sample-specific barcode which is the same as the sequence at the 5' end of the probe, and providing a free primer which is complementary to the sequence at the 3' end of the probe.

In the present application, after contacting the target molecule in the sample with the probe and before attaching the probe with the sample-specific barcode, providing a solid support attached with at least one PCR tag, where each of the PCR tags includes a sample-specific barcode and a PCR handle sequence at the 3' end of the sample-specific barcode which is the same as the sequence at the 3' end of the probe, and providing a free primer which is complementary to the sequence at the 5' end of the probe

In the present application, the barcoded probe can be formed by PCR. For example, the free primer is complemented to and extended with the 3' end sequence of the probe, and the extended sequence is complemented to and extended with the sequence of the PCR handle in the PCR tag to obtain the barcoded probe. For example, the extended sequence is complementary to the sequence of the PCR handle by 1% or more, 2% or more, 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, 99% or more. The remaining non-complementary nucleotides may be clustered or dispersed with the complementary nucleotides and need not be adjacent to each other or to the complementary nucleotides. For example, polynucleotides may hybridize on one or more segments, such that the intermediate or adjacent segments are not involved in hybridization events (e.g., hairpin formation, "bumps", etc.).

In the present application, the discrete compartment also includes a PCR reagent. For example, the PCR reagent may be a PCR amplification reagent. For example, at least a part of probes that bind the target molecule derived from the single cell can be released from the single cell in the discrete compartment to the outside of the cell, including at least 5%, at least 10%, at least 15%, at least 20%, at least 25%, at least 30%, at least 35%, at least 40%, at least 45% of the probes that bind the target molecule derived from the single cell are released from the single cell in the discrete compartment to the outside of the cell. For example, the step of releasing at least a part of probes that bind the target molecule derived from the single cell from the single cell in the discrete compartment to the outside of the cell may include contacting the cell with a lysing reagent to release the contents of the cell in the discrete compartment. The lysing reagent may include biologically active reagents, such as lysozymes, achromatic peptidases, lysostaphins, thioglycosidases, kitalases, lyticases, and other commercially available lysozymes, which are used to lyse different cell types (such as gram positive or negative bacteria, plants, yeasts, mammals, etc.). For example, cells can also be lysed using a surfactant-based lysing solution, for example, the lysing solution can include nonionic surfactants such as TritonX-100 and Tween 20. For example, the lysing solution may include ionic surfactants such as sodium dodecyl sarcosinate and sodium dodecyl sulfate (SDS). For example, other available lysing methods (such as electroporation, thermal, acoustic or mechanical cell destruction) may also be used.

In the present application, the barcoded probe can be formed by split pool synthesis. For example, the probe is co-dispensed into a discrete compartment with two or more of the PCR tags, the probe, and the primer, and the two or more PCR tags are different, such that the barcoded probe includes two or more sample-specific barcodes. For example, the probe is co-dispensed into discrete compartments with 3 or more, 4 or more, 5 or more, 6 or more, 7 or more or 8 or more of the PCR tags, the probe, and the primers, such that the barcoded probe includes 3 or more, 4 or more, 5 or more, 6 or more, 7 or more or 8 or more sample-specific barcodes.

In the present application, after contacting the target molecule in the sample with the probe and before attaching the probe with the sample-specific barcode, providing a solid support attached with at least one oligonucleotide tag where the oligonucleotide tag can be a single-stranded nucleic acid, and each oligonucleotide tag can contain a sample-specific barcode and a hybridization sequence at the 3' end of the sample-specific barcode. The probe can contain a first strand and a second strand, and the first strand contains a target molecule binding domain. For example, the second strand includes a second portion complementary to the oligonucleotide adapter in the probe, the first and second strands forming a partially double-stranded structure, and the second strand includes a first portion complementary to a hybridization sequence at the 3' end of the sample-specific barcode in the oligonucleotide tag. For example, the probe is attached to the solid support to which at least one oligonucleotide tag is attached in a discrete compartment to obtain a barcoded probe. In the present application, the method can further include the complementary hybridization of the first portion of the second strand in the probe with the hybridization sequence of the oligonucleotide tag, and the ligation of the hybridization sequence of the oligonucleotide tag with the probe to generate the barcoded probe. With respect to the hybridization, the conditions applicable to hybridization between two nucleic acids depend on the length and complementarity of the nucleic acid, which is well known in this field. The greater the degree of complementarity between the two nucleotide sequences, the greater the value of the unwinding temperature (Tm) of the hybrid of nucleic acids with these complementary sequences. In the present application, after the target molecule in the sample is in contact with the probe and before the probe is attached to a sample-specific bar code, a solid support with at least one oligonucleotide tag is provided, wherein each oligonucleotide tag includes a first strand and a second strand, the first strand includes a sample-specific bar code and a hybridization sequence at the 3' end of the sample-specific barcode, and the second strand includes a first portion complementary to the hybridization sequence of the first strand and a second portion complementary to the oligonucleotide adapter in the probe. The first and second strands forming a partially double-stranded structure, and the solid support attached to at least one oligonucleotide tag is connected to the probe in a discrete compartment to obtain a barcoded probe.

In the present application, a barcoded probe can be produced by linking the oligonucleotide tag with the probe. For example, the oligonucleotide tag may include a first strand including a sample-specific barcode and a hybridization sequence at the 3' end of the sample-specific barcode, the second strand including a first portion complementary to the hybridization sequence of the first strand and a second portion complementary to the sequence in the oligonucleotide adapter in the probe, and the first and second strands forming a partially double-stranded structure, the probe may be a single-stranded nucleic acid, the hybridization sequence of the first strand of the oligonucleotide tag being linked to the sequence in the oligonucleotide adapter in the probe, thereby producing the barcoded probe. For example, hybridizing the second portion of the second strand of the oligonucleotide tag with a sequence in the oligonucleotide adapter in the probe, and linking the hybridized sequence of the first strand of the oligonucleotide tag with a sequence in the oligonucleotide adapter in the probe, thereby producing the barcoded probe. With respect to the hybridization the conditions suitable for hybridization between two nucleic acids depend on the length and degree of complementarity of the nucleic acids which are well known in the art. The greater the degree of complementarity between two nucleotide sequences, the greater the value of the melting temperature (Tm) of the hybrids of nucleic acids having these complementary sequences.

For example, the length of the second portion of the second strand of the oligonucleotide tag is sufficient for it to form a double-stranded structure with its complementary sequence (the oligonucleotide adapter sequence or part thereof attached to the target nucleic acid).

For example, the length of the second portion of the second strand of the oligonucleotide tag may be 1 nucleotide or more, 2 nucleotides or more, 3 nucleotides or more, 5 nucleotides or more, 8 nucleotides or more, 10 nucleotides or more, 12 nucleotides or more, 15 nucleotides or more, 20 nucleotides or more, 22 nucleotides or more, 25 nucleotides or more, or 30 nucleotides or more.

For example, the hybridization does not exclude possible mismatches between bases. For example, the sequence of the first portion of the second strand or the second portion of the second strand need not be 100% complementary to the sequence of its hybridized sequence. For example, it can be 60% or more, 65% or more, 70% or more, 75% or more, 80% or more, 85% or more, 90% or more, 95% or more, 98% or more, 99% or more, 99.5% or more complementary. The remaining non-complementary nucleotides may be clustered or dispersed with the complementary nucleotides and need not be adjacent to each other or to the complementary nucleotides. For example, polynucleotides may hybridize on one or more segments such that intermediate or adjacent segments are not involved in hybridization events (e.g., hairpin formation, "bumps", etc.).

For example, an oligonucleotide tag is linked to the probe using a ligation reaction. The ligation may include binding two nucleic acid segments together by catalyzing the formation of a phosphodiester bond, such as the hybridization sequence of the first strand of the oligonucleotide tag and the sequence in the oligonucleotide adapter in the probe. The ligation reaction may include DNA ligases such as Escherichia coli DNA ligase, T4 DNA ligase, T7 DNA ligase, mammalian ligase (e.g., DNA ligase I, DNA ligase III, DNA ligase IV), thermostable ligase, etc. T4 DNA ligase can link segments containing DNA, oligonucleotides, RNA and RNA-DNA hybrids. The ligation reaction may not include DNA ligase, but a substitute, such as topoisomerase, is used. High concentration of DNA ligase and PEG can realize rapid ligation. In order to select a favorable temperature for the ligation reaction, the optimal temperature of the DNA ligase (e.g., 37°C) and the melting temperature of the DNA to be ligated can be considered. The probe and the barcoded solid support may be suspended in a suitable buffer to minimize ionic interaction that may affect the ligation.

For example, it includes releasing at least a portion of the probe from the single cell in the discrete compartment to the outside of the cell and linking the released probe with the oligonucleotide tag to produce a barcoded probe. For example, the step of releasing at least a portion of the probe from the single cell in the discrete compartment to the outside of the cell may include contacting the cell with a lysing reagent to release the contents of the cell in the discrete compartment. The lysing reagent may include biologically active reagents, such as lysozymes, achromatic peptidases, lysostaphins, thioglycosidases, kitalases, lyticases, and other commercially available lysozymes, which are used to lyse different cell types (such as gram positive or negative bacteria, plants, yeasts, mammals, etc.). For example, cells can also be lysed using a surfactant-based lysing solution, for example, the lysing solution can include nonionic surfactants such as TritonX-100 and Tween 20. For example, the lysing solution may include ionic surfactants such as sodium dodecyl sarcosinate and sodium dodecyl sulfate (SDS). For example, other available lysing methods (such as electroporation, thermal, acoustic or mechanical cell destruction) may also be used.

For example, releasing at least a portion of the probe from the single cell in the discrete compartment to the outside of the cell may include releasing at least 5%, at least 10%, at least 15%, at least 20%, at least 25%, at least 30%, at least 35%, at least 40%, at least 45% of the probe from the single cell in the discrete compartment to the outside of the cell.

For example, it includes allowing at least a portion of the oligonucleotide tag released from the solid support to enter the single cell and linking with the probe to produce a barcoded probe.

For example, the step of allowing at least a portion of the oligonucleotide tags released from the solid support to enter the single cell may include allowing at least 25%, at least 30%, at least 35%, at least 40%, at least 50%, at least 55%, at least 60%, at least 70%, at least 75%, at least 75% of the oligonucleotide tags to enter the single cell.

For example, the oligonucleotide tag can be releasably attached to the solid support. For example, an oligonucleotide tag releasable, cleavable, or reversibly attached to the solid support includes an oligonucleotide tag released or releasable by cleavage/destruction of a link between an oligonucleotide tag molecule and the solid support, or an oligonucleotide tag released by degradation of the solid support itself such that the oligonucleotide tag can be accessed or accessible by other agents, or both.

For example, the acrydite part linked with the solid support precursor, another substance linked with the solid support precursor, or the precursor itself contains unstable bonds, such as chemical, thermal, or photosensitive bonds, such as disulfide bonds, UV sensitive bonds, etc. The unstable bond may reversibly link (covalently link) a substance (e.g., an oligonucleotide tag) to a solid support. For example, thermally unstable bonds may include attachment based on nucleic acid hybridization (e.g., when the oligonucleotide hybridizes with a complementary sequence attached to a solid support) such that the pyrolysis chain of the hybrid releases the oligonucleotide from the solid support (or bead), for example, a sequence containing an oligonucleotide tag. In addition, the addition of various types of unstable bonds to a gel solid support may result in the production of a solid support capable of responding to different stimuli. Each type of unstable bond may be sensitive to an associated stimulus (e.g., chemical stimulus, light, temperature, etc.) such that the release of a substance attached to the solid support by each unstable bond may be controlled by applying an appropriate stimulus. For example, another substance containing unstable bonds can be attached to the gel solid support after the gel solid support is formed by the activation functional group of the gel bead. Reagents (with associated activatable groups) releasably attached to a solid support or otherwise arranged in discrete compartments may be provided such that the activatable groups may react with the desired reagent upon delivery to the desired set of reagents (e.g., by co-dispensing). Such activatable groups include cage groups, removable blocking or protecting groups, such as photo-unstable groups, thermally unstable groups, or chemically removable groups. In addition to thermally cleavable bonds, disulfide bonds, and UV-sensitive bonds, other non-limiting examples of unstable bonds that may be coupled to precursors or solid supports include ester bonds (e.g., cleavable by acid, base, or hydroxylamine), o-diol bonds (e.g., cleavable by sodium periodate), Diels-Alder bonds (e.g., cleavable by heat), sulfone bonds (e.g., cleavable by base), silyl ether bonds (e.g., cleavable by acid), glycoside bonds (e.g., cleavable by amylase), peptide bonds (e.g., cleavable by protease), or phosphodiester bonds (e.g., cleavable by nuclease (DNase).

For example, the oligonucleotide tag is directly or indirectly attached to the solid support through the 5' end of its first strand.

For example, in the barcoded probe, the probe is located at the 3' end of the sample-specific barcode. For example, the probe may be directly linked to the 3' end of the sample-specific barcode; for example, the probe may not be directly linked to the 3' end of the sample-specific barcode, and any other nucleotide sequence may be present between the probe and the sample-specific barcode.

For example, in the barcoded probe, the probe is located at the 5' end of the sample-specific barcode. For example, the probe may be directly linked to the 5' end of the sample-specific barcode; for example, the probe may not be directly linked to the 5' end of the sample-specific barcode, and any other nucleotide sequence may exist between the probe and the sample-specific barcode.

For example, the barcoded probe can be amplified. For example, the barcoded probe is released from the discrete compartment, and the amplification is performed after the barcoded probe is released from the discrete compartment. For example, after the barcoded probe is released from the discrete compartment, further chemical or enzymatic modification may be performed, followed by amplification.

For example, amplification primers are used in the amplification. For example, the amplification may also include further modification of the barcoded target nucleic acid so that it also has an immobilized sequence on the other side that can be used for PCR amplification. For example, the modification may include reverse transcription strand conversion, second strand synthesis, and terminal transferase reaction.

For example, the amplification primers may also include universal primers.

For example, the amplification primers are used in the amplification, and the amplification primers may include random guide sequences. The random guide sequence includes random primers that can exhibit quadruple degeneracy at each position. For example, random primers include any nucleic acid primers known in the art with various random sequence lengths. For example, a random primer may include a random sequence of 3, 4, 5, 6, 7, 8, 10, 11, 12, 13, 14, 15, 16, 17, 18, 19, 20 or more nucleotides in length. For example, a plurality of random primers may include random primers with different lengths. For example, a plurality of random primers may include random primers having equal lengths. For example, the plurality of random objects may include random sequences of about 5 to about 18 nucleotides in length. For example, a plurality of random objects include random hexamer. The random hexamer is commercially available and widely used in amplification reactions, such as multiple displacement amplification (MDA), for example, the REPLI-g genome-wide amplification kit (QIAGEN, Valencia, CA). Random primers of any suitable length can be used in the methods and compositions described herein.

For example, the amplification includes at least partially hybridizing the random pilot sequence with the barcoded probe and extending the random pilot sequence in a template-oriented manner.

In the present application, the target molecule may include DNA derived from the single cell. For example, the DNA includes genomic DNA, open chromatin DNA, protein-binding DNA regions, and/or exogenous nucleic acids bound to proteins, lipids, and/or small molecule compounds capable of binding to target molecules within cells.

In the present application, the DNA derived from a single cell can be fragmented. For example, the probe that binds to the target molecule may be produced after or during the fragmentation. For example, the fragmentation may include using ultrasonic cleavage followed by adding a sequence including the oligonucleotide adapter to the cleaved DNA to obtain a probe that binds to a target molecule.

For example, the fragmentation may include using a DNA endonuclease, an exonuclease interruption, and then adding a sequence including the oligonucleotide adapter to the cleaved DNA to obtain a probe that binds to a target molecule. For example, the nucleotide adapter sequence includes a transposon terminal sequence. For example, the transposon terminal sequence is a Tn5 or a modified Tn5 transposon terminal sequence. For example, the transposon terminal sequence is the Mu transposon terminal sequence. For example, the Tn5 or modified Tn5 transposon terminal sequence or Mu transposon terminal sequence may contain 15 to 25 nucleotides, for example, 16 nucleotides, 17 nucleotides, 18 nucleotides, 19 nucleotides, 20 nucleotides, 21 nucleotides, 22 nucleotides, 23 nucleotides, 24 nucleotides.

For example, the fragmentation may include integrating a sequence including the oligonucleotide adapter into the DNA using a transposase-nucleic acid complex and releasing the transposase to obtain the attached target nucleic acid. For example, the transposase-nucleic acid complex may include a transposase and a transposon-terminal nucleic acid molecule, wherein the transposon-terminal nucleic acid molecule may include the oligonucleotide adapter sequence. For example, the transposase may include Tn5. For example, the DNA may include a DNA region that binds to a protein, and the transposase-nucleic acid complex may also include a part that directly or indirectly recognizes the protein. For example, the part that directly or indirectly recognizes the protein may include one or more of antibodies that specifically bind to the protein and protein A or protein G.

In the present application, a barcoded probe can be produced by linking the oligonucleotide tag with the probe. For example, the oligonucleotide tag may include a first strand including a sample-specific barcode and a hybridization sequence at the 3' end of the sample-specific barcode, the second strand including a first portion complementary to the hybridization sequence of the first strand and a second portion complementary to the sequence in the oligonucleotide adapter in the probe, and the first and second strands forming a partially double-stranded structure, the probe may be a single-stranded nucleic acid, the hybridization sequence of the first strand of the oligonucleotide tag being linked to the sequence in the oligonucleotide adapter in the probe, thereby producing the barcoded probe. For example, hybridizing the second portion of the second strand of the oligonucleotide tag with a sequence in the oligonucleotide adapter in the probe, and linking the hybridized sequence of the first strand of the oligonucleotide tag with a sequence in the oligonucleotide adapter in the probe, thereby producing the barcoded probe. With respect to the hybridization the conditions suitable for hybridization between two nucleic acids depend on the length and degree of complementarity of the nucleic acids which are well known in the art. The greater the degree of complementarity between two nucleotide sequences, the greater the value of the melting temperature (Tm) of the hybrids of nucleic acids having these complementary sequences.

For example, the length of the second portion of the second strand of the oligonucleotide tag is sufficient for it to form a double-stranded structure with its complementary sequence (the oligonucleotide adapter sequence or part thereof attached to the target nucleic acid).

For example, the length of the second portion of the second strand of the oligonucleotide tag may be 1 nucleotide or more, 2 nucleotides or more, 3 nucleotides or more, 5 nucleotides or more, 8 nucleotides or more, 10 nucleotides or more, 12 nucleotides or more, 15 nucleotides or more, 20 nucleotides or more, 22 nucleotides or more, 25 nucleotides or more, or 30 nucleotides or more.

For example, the hybridization does not exclude possible mismatches between bases. For example, the sequence of the first portion of the second strand or the second portion of the second strand need not be 100% complementary to the sequence of its hybridized sequence. For example, it can be 60% or more, 65% or more, 70% or more, 75% or more, 80% or more, 85% or more, 90% or more, 95% or more, 98% or more, 99% or more, 99.5% or more complementary. The remaining non-complementary nucleotides may be clustered or dispersed with the complementary nucleotides and need not be adjacent to each other or to the complementary nucleotides. For example, polynucleotides may hybridize on one or more segments such that intermediate or adjacent segments are not involved in hybridization events (e.g., hairpin formation, "bumps", etc.).

For example, an oligonucleotide tag is linked to the probe using a ligation reaction. The ligation may include binding two nucleic acid segments together by catalyzing the formation of a phosphodiester bond, for example, the hybridization sequence of the first strand of the oligonucleotide tag and the sequence in the oligonucleotide adapter in the probe. The ligation reaction may include DNA ligases such as Escherichia coli DNA ligase, T4 DNA ligase, T7 DNA ligase, mammalian ligase (e.g., DNA ligase I, DNA ligase III, DNA ligase IV), thermostable ligase, etc. T4 DNA ligase can link segments containing DNA, oligonucleotides, RNA and RNA-DNA hybrids. The ligation reaction may not include DNA ligase, but a substitute, such as topoisomerase, is used. High concentration of DNA ligase and PEG can realize rapid ligation. In order to select a favorable temperature for the ligation reaction, the optimal temperature of the DNA ligase (e.g., 37°C) and the melting temperature of the DNA to be ligated can be considered. The probe and the barcoded solid support may be suspended in a suitable buffer to minimize ionic interaction that may affect the ligation.

For example, it includes releasing at least a portion of the probe from the single cell in the discrete compartment to the outside of the cell and linking the released probe with an oligonucleotide tag to produce a barcoded probe. For example, the step of releasing at least a portion of the probe from the single cell in the discrete compartment to the outside of the cell may include contacting the cell with a lysing reagent to release the contents of the cell in the discrete compartment. The lysing reagent may include biologically active reagents, such as lysozymes, achromatic peptidases, lysostaphins, thioglycosidases, kitalases, lyticases, and other commercially available lysozymes, which are used to lyse different cell types (such as gram positive or negative bacteria, plants, yeasts, mammals, etc.). For example, cells can also be lysed using a surfactant-based lysing solution, for example, the lysing solution can include nonionic surfactants such as TritonX-100 and Tween 20. For example, the lysing solution may include ionic surfactants such as sodium dodecyl sarcosinate and sodium dodecyl sulfate (SDS). For example, other available lysing methods (such as electroporation, thermal, acoustic or mechanical cell destruction) may also be used.

For example, releasing at least a portion of the probe from the single cell in the discrete compartment to the outside of the cell may include releasing at least 5%, at least 10%, at least 15%, at least 20%, at least 25%, at least 30%, at least 35%, at least 40%, at least 45% of the probe from the single cell in the discrete compartment to the outside of the cell.

For example, it includes allowing at least a portion of the oligonucleotide tag released from the solid support to enter the single cell and linking with the probe to produce a barcoded probe.

For example, the step of allowing at least a portion of the oligonucleotide tags released from the solid support to enter the single cell may include allowing at least 25%, at least 30%, at least 35%, at least 40%, at least 50%, at least 55%, at least 60%, at least 70%, at least 75%, at least 75% of the oligonucleotide tags to enter the single cell.

For example, the oligonucleotide tag can be releasably attached to the solid support. For example, an oligonucleotide tag releasable, cleavable, or reversibly attached to the solid support includes an oligonucleotide tag released or releasable by cleavage/destruction of a link between an oligonucleotide tag molecule and the solid support, or an oligonucleotide tag released by degradation of the solid support itself such that the oligonucleotide tag can be accessed or accessible by other agents, or both.

### Solid support

In the present application, the solid support may include beads. For example, the beads may be porous, non-porous and/or combinations thereof. For example, the beads may be solid semi-solid semi-fluid fluid and/or combinations thereof. For example, the beads may be soluble, breakable and/or degradable. For example, the beads may be non-degradable. For example, the beads may be gel beads. The gel beads may be hydrogel beads. Gel beads can be formed from molecular precursors, such as polymers or monomer substances. The semisolid beads may be liposome beads. The solid beads may contain metals, including iron oxide, gold and silver. For example, the beads may be silica beads. For example, the beads are magnetic beads. For example, the beads may be rigid. For example, the beads may be flexible and/or compressible.

For example, the beads may have any suitable shape. For example, the shape of the bead may include, but not limited to, spherical, non-spherical, oval, oblong, amorphous, circular, cylindrical, and variations thereof.

For example, the beads may have a uniform size or a non-uniform size. For example, the diameter of the beads may be at least about 10 nm, 100 nm, 500 nm, 1 µm, 5 µm, 10 µm, 20 µm, 30 µm, 40 µm, 50 µm, 60 µm, 70 µm, 80 µm, 90 µm, 100 µm, 250 µm, 500 µm, 1mm or greater. For example, the diameter of the beads may be less than about 10 nm, 100 nm, 500 nm, 1 µm, 5 µm, 10 µm, 20 µm, 30 µm, 40 µm, 50 µm, 60 µm, 70 µm, 80 µm, 90 µm, 100 µm, 250 µm, 500 µm, 1 mm or less. For example, the diameter of the beads may be in the range of about 40-75 µm, 30-75 µm, 20-75 µm, 40-85 µm, 40-95 µm, 20-100 µm, 10-100 µm, 1-100 µm, 20-250 µm, or 20-500 µm.

For example, the beads may be provided in the form of a population of beads or a plurality of beads having a relatively monodisperse size distribution. Maintaining relatively consistent bead characteristics (e.g., size) may facilitate overall consistency where a relatively consistent amount of reagent is required to be provided in a discrete compartment. In particular, the beads described herein may have size distributions with a coefficient of variation of their cross-sectional dimensions of less than 50%, less than 40%, less than 30%, less than 20%, and for example less than 15%, less than 10%, less than 5%, or less.

For example, beads may contain natural and/or synthetic materials. For example, beads may contain natural polymers, synthetic polymers, or natural and synthetic polymers. Natural polymers may include proteins and sugars, such as deoxyribonucleic acid, rubber, cellulose, starch (e.g., amylose, amylopectin), proteins, enzymes, polysaccharides, silk, polyhydroxyalkanoates, chitosan, glucan, collagen, carrageenan, plantaginis ovata, gum arabic, agar, gelatin, shellac, sycamore gum, xanthan gum, corn sugar gum, guar gum, sycamore gum, agarose, alginate, alginate or their natural polymers. Synthetic polymers may include acrylic, nylon, siloxane, spandex, viscose rayon, polycarboxylic acid, polyvinyl acetate, polyacrylamide, polyacrylate, polyethylene glycol, polyurethane, polylactic acid, silicon dioxide, polystyrene, polyacrylonitrile, polybutadiene, polycarbonate, polyethylene, polyethylene, polyethylene terephthalate, polychlorotrifluoroethylene, polyethylene oxide, polyethylene terephthalate, polyisobutylene, polymethyl methacrylate, polyoxymethylene, polypropylene, polystyrene, polytetrafluoroethylene, polyvinyl alcohol, polyvinyl chloride, polyvinylidene chloride, polyvinylidene fluoride, polyvinylidene fluoride, polyvinylidene fluoride and/or their combinations ( for example, copolymers ). Beads can also be formed by materials other than polymers, such as lipids, micelles, ceramics, glass ceramics, material composites, metals, other inorganic materials, etc.

For example, the beads may contain molecular precursors (e.g., monomers or polymers) which may form a polymer network by polymerization of the molecular precursors. For example, the precursor may be a substance that has been polymerized which can be further polymerized, for example, by chemical crosslinking. For example, the precursor may include one or more of an acrylamide or methacrylamide monomer oligomer or polymer. For example, the beads may include a prepolymer which is an oligomer capable of further polymerization. For example, polyurethane beads may be prepared using a prepolymer. For example, the beads may contain separate polymers that can be further polymerized together. For example, beads may be produced by polymerization of different precursors such that they include mixed polymers copolymers and/or block copolymers. For example, beads may contain covalent or ionic bonds between polymer precursors (e.g., monomers, oligomers, linear polymers), nucleic acid molecules (e.g., oligonucleotides), primers, and other entities. For example, the covalent bond may be a carbon-carbon bond, a thioether bond, or a carbon-heteroatom bond.

For example, the crosslinking may be permanent or reversible depending on the particular crosslinking agent used. Reversible crosslinking allows the polymer to be linearized or dissociated under appropriate conditions. For example, reversible crosslinking may also allow the binding substance to reversibly attach to the bead surface. For example, the crosslinking agent may form a disulfide bond. For example, the chemical crosslinking agent forming disulfide bonds may be cystamine or modified cystamine.

For example, disulfide bonds may be formed between bead-incorporated molecular precursor units (e.g., monomers, oligomers, or linear polymers) or between a precursor and a nucleic acid molecule (e.g., oligonucleotide). For example, cystamine (including modified cystamine) is an organic reagent containing disulfide bonds that can be used as a crosslinking agent between individual monomers of beads or polymer precursors. The polyacrylamide may be polymerized in the presence of cystamine or a substance including cystamine (e.g., modified cystamine) to produce polyacrylamide gel beads including disulfide bonds (e.g., chemically degradable beads including chemically reducible crosslinking agents). Disulfide bonds may allow degradation or lysing of beads when exposed to reducing agents.

For example, chitosan (linear polysaccharide polymer) can be crosslinked with glutaraldehyde through hydrophilic chains to form beads. Crosslinking of chitosan polymers can be achieved by chemical reactions induced by heat, pressure, pH changes and/or radiation.

For example, the bead can be macromolecules of single or mixed monomers polymerized by various monomers such as agarose, polyenamide and PEG, or macromolecular gels such as chitin, hyaluronic acid and dextran, which are polymerized into gel beads with uniform size in microdroplets by using microfluidic droplet platform.

For example, the bead may include an acrydite part, which, in some aspects, may be used to attach one or more nucleic acid molecules (e.g., barcodes, barcode nucleic acid molecules, barcode oligonucleotides, primers, or other oligonucleotides) to the bead. For example, an acrydite part may refer to an acrydite analogue produced by the reaction of acrydite with one or more substances, such as the reaction of acrydite with other monomers and crosslinking agents during the polymerization reaction. The acrydite part may be modified to form a chemical bond with a substance to be attached, such as a nucleic acid molecule (e.g., sample-specific barcodes, barcoded probes, primers, or other oligonucleotides). The acrydite part may be modified with a mercaptan group capable of forming a disulfide bond or may be modified with a group that already contains a disulfide bond. A mercaptan or disulfide (via disulfide exchange) may be used as an anchor point for the substance to be attached, or another part of the acrydite part may be used for attachment. For example, the attachment may be reversible such that when the disulfide bond breaks (e.g., in the presence of a reducing agent) the attached substance is released from the beads. In other cases the acrydite part may contain reactive hydroxyl groups that may be used for attachment. In addition to disulfide bonds, other release modes may be included, such as UV photoinduced release, or enzyme release may be used.

### Discrete compartment

The present application provides a device for co-dispensing a solid support (e.g., beads) with a sample, for example, for co-dispensing sample components and beads to the same discrete compartment. For example, the probe and the solid support attached with at least one oligonucleotide tag or PCR tag are co-dispensed into the discrete compartment.

For example, the discrete compartment may include pores or microdroplets. For example, the probe and the solid support attached with at least one oligonucleotide tag are co-dispensed into the pores or microdroplets. For example, the pores may include an upper sample pore of a cell culture plate or any other container pores capable of cooperating with the device and adapted for co-dispensing. For example, the pores may include the pores of a 96-well plate. For example, the discrete compartments are microdroplets. For example, each of the discrete compartments includes at most a probe that binds to the target molecule derived from a single cell. For example, the target molecule is located in a single cell or nucleus. For example, the target molecule binding to a single cell is co-dispensed with the solid support attached with at least one oligonucleotide tag or PCR tag into the discrete compartment by using a microfluidic device.

For example, the discrete compartment (e.g., droplets or pores) contain single cells and are processed according to the methods of the present application. For example, discrete compartments contain single cells and/or single nuclei. Single cells and/or single nuclei may be dispensed and processed according to the methods of the present application. For example, a single cell nucleus can be a constituent part of a cell. For example, discrete compartments contain chromatin from a single cell or a single nucleus (e.g., a single chromosome or other part of the genome) and are dispensed and processed according to the methods of the present application.

For example, a ligase is also included in the discrete compartment, and the ligase enables the oligonucleotide tag to be linked to a probe that has been bound to the target molecule. The discrete compartment includes, but not limited to, a ligase, and may also include other desired enzymes. For example, DNA polymerases, DNA endonucleases, DNA exonucleases, terminal transferases, and enzymes capable of making the oligonucleotide tags released from the solid support pH sensitive to photosensitive enzyme activity. The ligase includes, but not limited to, T4 ligase, and may also include, e.g., *Escherichia coli* DNA ligase, T4 DNA ligase, T7 DNA ligase, mammalian ligase (e.g., DNA ligase I, DNA ligase III, DNA ligase IV), thermostable ligase, etc.

In the present application, the microfluidic device may be a droplet generator. For example, a solid support to which at least one oligonucleotide tag is attached may be combined with a sample (for example, a sample containing a target nucleic acid) by forming aqueous microdroplets containing both the solid support to which at least one oligonucleotide tag is attached and a sample using a microfluidic device. The water-bome small microdroplets serve as discrete compartments. The aqueous droplet may be an aqueous core surrounded by an oil phase, for example, an aqueous droplet within a water-in-oil emulsion. The aqueous droplet may contain one or more solid supports to which at least one oligonucleotide tag is attached, a sample, an amplification reagent, and a reducing agent. For example, the aqueous droplet may include one or more of water, nuclease-free water, solid support to which at least one oligonucleotide tag is attached, acetonitrile, solid support, gel solid support, polymer precursor, polymer monomer, polyacrylamide monomer, acrylamide monomer, degradable crosslinker, non-degradable crosslinker, disulfide bond, acrydite part, PCR reagent, cell, nucleus, chloroplast, mitochondria, ribosome, primer, polymerase, cell barcode, polynucleotide, oligonucleotide, DNA, RNA, peptide polynucleotide, complementary DNA (cDNA), double-chained DNA (dsDNA), single-stranded DNA (ssDNA), Mitochondrial DNA, ribosomal RNA, viral DNA, bacterial DNA, mtDNA (mitochondrial DNA), mRNA, rRNA, tRNA, nRNA, siRNA, snRNA, snoRNA, scaRNA, microRNA, dsRNA, probes, dyes, organic compounds, emulsifiers, surfactants, stabilizers, polymers, aptamers, reducing agents, initiators, biotin markers, fluorophore, buffers, acidic solutions, alkaline solutions, light-sensitive enzymes, pH-sensitive enzymes, aqueous buffers, oils, salts, detergents, ionic detergents, nonionic detergents, etc. In a word, the composition of the aqueous microdroplets will change according to the specific treatment requirements.

Aqueous microdroplets may have uniform or non-uniform sizes. For example, the diameter of the aqueous droplet may be about 1 µm, 5 µm, 10 µm, 20 µm, 30 µm, 40 µm, 45 µm, 50 µm, 60 µm, 65 µm, 70 µm, 75 µm, 80 µm, 90 µm, 100 µm, 250 µm, 500 µm or 1mm. For example, the fluid droplet may have a diameter of at least about 1 µm, 5 µm, 10 µm, 20 µm, 30 µm, 40 µm, 45 µm, 50 µm, 60 µm, 65 µm, 70 µm, 75 µm, 80 µm, 90 µm, 100 µm, 250 µm, 500 µm, 1mm or greater. For example, a fluid droplet may have a diameter less than about 1 µm, 5 µm, 10 µm, 20 µm, 30 µm, 40 µm, 45 µm, 50 µm, 60 µm, 65 µm, 70 µm, 75 µm, 80 µm, 90 µm, 100 µm, 250 µm, 500 µm, or 1mm. For example, a fluid droplet may have a diameter in the range of about 40-75 µm, 30-75 µm, 20-75 µm, 40-85 µm, 40-95 µm, 20-100 µm, 10-100 µm, 1-100 µm, 20-250 µm, or 20-500 µm.

### Combination and kit

The present application also provides a combination including: 1) two or more probes including a target molecule binding domain and a probe identity tag; 2) a plurality of solid supports, each of which is attached to a solid support of at least one PCR tag, wherein each of the PCR tags includes a sample-specific barcode and a PCR handle sequence at the 3' end of the sample-specific barcode, the PCR handle sequence being same to the probe identity tag sequence and providing free primers and PCR reagents, the primers complementing the sequence of the target molecule binding domain in the probe.

The present application also provides a kit including the combination described in the present application. For example, the kit may also include a transposase. For example, the kit further includes at least one of a nucleic acid amplification agent, a reverse transcription agent, an immobilizing agent, a permeating agent, a linking agent, and a lysis agent.

Without being limited by any theory the following examples are only intended to illustrate the compositions kits methods and uses of the present application and are not intended to limit the scope of the present invention.

### Embodiments

### EXAMPLE 1 Primer design

1.1 The primers were designed by common primer design program. The following primers were designed by primer 3. In the transcriptome gene model of target species, target genes were selected, and 10 pairs of primers are designed for each gene. According to the adjacent positions in the gene, the upstream and downstream primers with a length of 25 bp were respectively designed to ensure their specificity.

### Specific examples:

Upstream primer for each pair of primers: ATCCACGTGCTTGAG (SEQ ID NO: 1)-UMI-probe sequence
Downstream primer for each pair of primers: Probe-AGATCGGAAGAGCACACGTCTG (SEQ ID NO: 9)
where, the sequence of UMI is nnnnnnn (random sequence).
1.2 Synthesis of upstream primers
1.2.1 A set of probe primers was first parallelly synthesized with Twist Bioscience.

The sequence of the probe primer set is:
ATCCACGTGCTTGAG (SEQ ID NO: 1)-nnnnnnn-Probe-GATCACTCTGCGTTGATACCAC (SEQ ID NO: 10)
1.2.2 A library of upstream primers was amplified by PCR using the following primers.
   Primer 1: biotin-ATCCACGTGCTTGAG (SEQ ID NO: 1)
   Primer 2: GTGGTATCAACGCAGAGTGATC (SEQ ID NO: 2)
1.2.3 After that, the PCR product was purified and digested with DpnII to remove the downstream sequence of the probe.
1.2. 4 Binding with streptavidin (SA) magnetic beads, the sequence is as below:
   Magnetic beads-SA-Biotin-ATCCACGTGCTTGAG (SEQ ID NO: 1) nnnnnnnn-probe-
1.2.5 Then denaturing with 0.1 M NaOH to remove PCR complementary chains.
1.2.6 Then heating at 100°C to release the forward probe primer bound to streptavidin magnetic beads.
Biotin-ATCCACGTGCTTGAG (SEQ ID NO: 1) nnnnnnnn-probe-
1.3 Synthesis of downstream primers
1.3.1 A set of probe primers was parallelly synthesized with Twist Bioscience.

The sequence of the probe primer set is:
CTTCCGATCTGGTCTCG (SEQ ID NO: 3)-Probe-AGATCGGAAGAGCACACGTCTG (SEQ ID NO: 9)
1.3.2 A library of downstream primers was amplified by PCR using the following primers.
Primer 3: Biotin-CTTCCGATCTGGTCTCG (SEQ ID NO: 3)
Primer 4: Biotin-CAGACGTGTGCTCTTCCGATCT (SEQ ID NO: 4)
1.3.3 PCR products were bound with streptavidin magnetic beads and digested with BsaI-HF enzyme to remove redundant primer sequences.
1.3.4 Then denaturation by heating at 95°C to obtain the final primer, as shown below.
Probe-AGATCGGAAGAGCACACGTCTG (SEQ ID NO: 9)

### EXAMPLE 2 Sample preparation, hybridization of probe with sample

2.1 First, the cells were immobilized with 4% formaldehyde in PBS at room temperature for 10 min, then dehydrated and stored in 100% ethanol at -20°C.
2.2 After that, a hybridization solution was prepared, and the composition of the hybridization solution was 10% formamide + 2× sodium citrate buffer (SSC) + 10% sodium dextran sulfate.
2.3 The cells were pre-hybridized in the hybridization solution for 1 hour at 42°C.
2.4 The upstream primer pool and downstream primer pool were added to the cells to a total concentration of 10 uM.
2.5 Then hybridization at 42°C for 12-24 hours.
2.6 The cells were washed twice with the hybridization solution.
2.7 The cells were washed twice with PBS containing 1% BSA.
2.8 The cells were re-suspended with 1×T4 ligase buffer and ligated with T4 ligase at room temperature for 1 hour.
2.9 The cells were washed twice with PBS containing 1% BSA.

### EXAMPLE 3 Labeling of single cells by ligation

3.1 Preparation of an oligonucleotide tag including a sample-specific barcode, which is immobilized on a solid support (e.g., beads).

The sequences attached to the solid support (e.g., beads) are as below: Bead-S-S-PCR adapterbarcode 1-linker 1-barcode 2-linker 2-barcode 3-linker handle (hybridization sequence)

Among them, the sequence of the PCR adapter is ACACTCTTTCCCTACACGACGCTCTTCCGATCT (SEQ ID NO: 5), Linker 1 is CGACTCACTACAGGG (SEQ ID NO: 6), and the sequence of Linker 2 is TCGGTGACACGATCG (SEQ ID NO: 7), the sequence of the linker handle is GACAGC. Barcode 1 = 96 sequences of 5bp base, Barcode 2 = 96 sequences of 5bp base, Barcode 3 = 96 sequences of 5bp base.

### 3.2 Synthesis of 3×96 sequences

3.2.1 PCR handle-96×barcode 1-linker 1, and 96 reverse complementary sequences of these sequences were synthesized;
3.2.1 Linker 1-96×barcode 2-linker 2, and 96 reverse complementary sequences of these sequences were synthesized;
3.2.3 Linker 2-96×barcode 3-PCR linker, and 96 reverse complementary sequences of these sequences were synthesized.

### 3.3 Synthesis of beads:

The following amino sequence was synthesized: 5'amine-S-S-ACACTCTTTCCCTACACGACGCTCTTCCGATCT (SEQ ID NO: 5) and 30 µm carboxyl modified beads (Knowledge & Benefit, KBsphere^{®}, www.kbspheres.com/productshow.asp?id=903).

Coupling reaction: bead + 50 mM EDC + 100 µm amino sequence, the amino sequence and carboxyl microspheres were coupled to obtain the following structure: bead-S-S-ACACTCTTTCCCTACACGACGCTCTTCCGATCT (SEQ ID NO: 5).

### 4) Attachment of tags

The synthesized beads were equally divided into 96-well plates, and then added with PCR handle-96×barcode 1-linker 1, respectively, for the first round of barcoding reaction. The reaction system and process are as follows: 10 µl of microspheres + 2 µl of BstI buffer + 1 µl of 10 µM dNTP + 1 µl of 100 µM PCR handle-96× barcode 1-linker 1, then hold at 95°C for 5 min and 60°C for 20 min; then 1 µl of BstI + 5 µl of H₂O were added and hold at 60°C for 60 min.

After the first round of barcoding reaction, all beads were collected, mixed, reacted at 95°C for 5 min to remove complementary strands, and washed to obtain the microspheres barcoded in the first round (96 × barcode 1-linker 1). Then, the beads were equally divided into 96-well plates, into which were added the linker 1-96×barcode 2-linker 2 (the second round) and the linker 2-96×barcode 3-PCR handle (the third round). The second and third rounds of barcoding reaction were carried out according to the system method of the first round to obtain the single-stranded beads with triple tags finally. After the beads were washed, they were annealed with the complementary sequence GCTCTGGCTGTC (SEQ ID NO: 8 (reverse sequence)) to form a partial double-stranded structure, finally obtaining the following beads attached with a partially double-stranded structure:
Beads-S-S-ACACTCTTTCCCTACACGACGCTCTTCCGATCT (SEQ ID NO: 5)-Barcode 1-CGACTCACTAGGG (SEQ ID NO: 6)-Barcode 2-TCGGTGACACGATCG (SEQ ID NO: 7)-Barcode 3-ATCCACCGTGCTTGAG (SEQ ID NO: 1).

### EXAMPLE 4 Labeling of single cells by droplet PCR

4.1 On a microfluidic device, the cells hybridized with the probe, the labelled beads and the PCR enzyme reagent are wrapped into microspheres with primers as below.
   Primer: ACACTCTTTCCCTACACGACGCTCTTCCGATCT (SEQ ID NO: 5)
   Primer: CAGACGTGTGCTCTTCCGATCT (SEQ ID NO: 4)
4.2 Perform the following PCR reaction in droplets
   Reaction system: at 95°C for 5 min; at 95°C for 10 s; at 55°C for 20 s; at 72°C for 30 s; 20 cycles.
4.3 All droplets were recovered and broken with perfluoro-octanol, and the aqueous phase was collected.
4.4 PCR products were recovered with 1.8 × magnetic beads, and the primers for ligation in each cell were added with cell barcodes, with the structure shown as below.
   That is, Read1-cell barcode-PCR adapter-UMI-upstream probe-downstream probe-read2
4.5 PCR was performed for the obtained products with Illumina Trueseq Index Primer, and a sequencing library was finally obtained.

### EXAMPLE 5 Bioinformatics analysis

5.1 The number of cells in the sample was identified by cell barcode.
5.2 The detected probe sequence was compared with the designed 50 bp upstream and downstream primer sequences, and for exact match, the absolute number of probes hybridizing to the target molecules in the cells was calculated based on the probe identity tag (UMI) data.
5.3 The average number of probes for all hybridization on each gene was calculated to obtain the expression value of each gene, and finally obtain the gene expression matrix for each cell.

### Analysis results

The test sequencing data of the present application is shown in the table below:

| Samples | 293 cell lines |
|---|---|
| Number of input reads | 21772642 |
| Average input read length | 50 +/-5bp |

| UNIQUE READS: | |
|---|---|
| Uniquely mapped reads number | 17932138 |
| Uniquely mapped reads% | 82.36% |
| Average mapped length | 50bp |
| % of read mapped to multiple loci | 9.04% |
| % of reads mapped to too many loci | 1.67% |

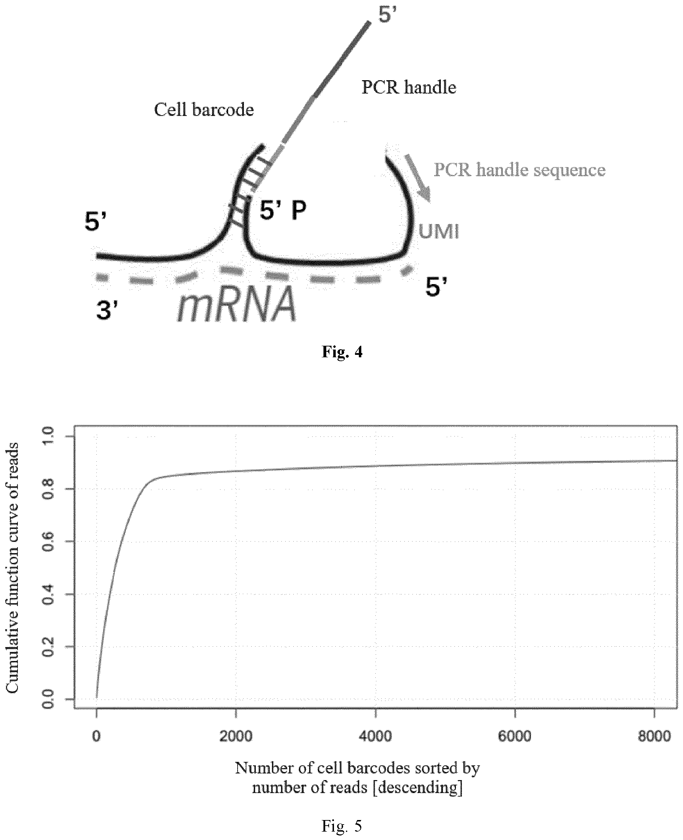

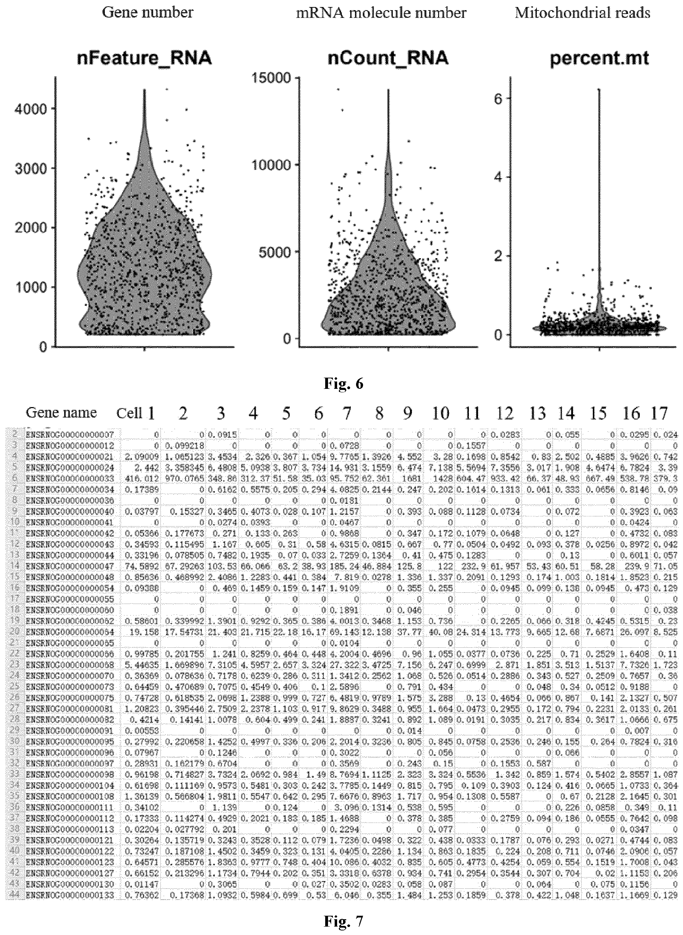

The prediction results of cell number in the present application are shown in Fig. 5. The detection results of gene number, mRNA molecule number and mitochondrial reads are shown in Fig. 6 and the table below.

| | |
|---|---|
| Samples | 293 cell lines |
| Cell Number (Genes > 200) | 948 |
| Median Genic Reads | 12609 |
| Median TRANSCRIPTS | 2165 |
| Median GENES | 1257 |
| Mean Genic Reads | 15709 |
| Mean TRANSCRIPTS | 2658 |
| Mean GENES | 1318 |

The detection results for an examplary single cell gene expression matrix in the present application are shown in Fig. 7. The results show that the analysis method of the present application can be used for high-throughput analysis of various omics information such as transcriptomics information of single cells.

The foregoing detailed description is provided by way of explanation and examples and is not intended to limit the scope of the appended claims. Various modifications of the embodiments enumerated herein will be apparent to those of ordinary skills in the art and are retained within the scope of the appended claims and equivalent embodiments thereof.

## Claims

1. A method for analyzing a target molecule from a sample, the method comprising:
contacting at least one target molecule in the sample with at least one probe, the probe comprises a target molecule binding domain, and the target molecule binding domain is capable of specifically recognizing the at least one target molecule in the sample;
attaching the probe to a sample-specific barcode to obtain a barcoded probe; and
determining the composition of the barcoded probe, and determining the presence and/or content of the target molecule in the sample from the composition of the barcoded probe.

2. The method of claim 1, wherein the target molecule comprises a protein, a nucleic acid molecule, a metabolite, and/or a lipid.

3. The method of claim 2, wherein the nucleic acid molecule comprises a DNA molecule and/or an RNA molecule.

4. The method of claim 3, wherein the DNA comprises cDNA.

5. The method according to any one of claims 3-4, wherein the RNA comprises cRNA, mRNA, miRNA, IncRNA, and/or eRNA.

6. The method according to any one of claims 1-5, wherein the sample comprises at least one cell.

7. The method according to any one of claims 1-6, wherein the sample is a single cell.

8. The method according to any one of claims 1-7, wherein the target molecule in the sample is one or more mRNA in the single cell.

9. The method according to any one of claims 6-8, wherein the cell is selected from the group consisting of a cell isolated from an organism, a cell cultured *in vitro,* a primary cell and a discrete cell from an explant.

10. The method according to any one of claims 6-9, wherein the cell is immobilized.

11. The method according to any one of claims 1-10, wherein the target molecule comprises a nucleic acid molecule and the target molecule binding domain in the probe comprises a single-stranded nucleic acid.

12. The method of claim 11, wherein the single-stranded nucleic acid comprises at least 10 nucleotides.

13. The method according to any one of claims 11-12, wherein the single-stranded nucleic acid comprises about 10-60 nucleotides.

14. The method according to any one of claims 11-13, wherein the target molecule binding domain in the probe is at least 50% complementary to the sequence of the target molecule.

15. The method according to any one of claims 11-14, wherein the composition of the target molecule is known.

16. The method according to any one of claims 11-15, wherein the target molecule is mRNA, and the nucleic acid sequence of the mRNA is known.

17. The method of claim 16, wherein the target molecule binding domain in the probe is designed according to the nucleic acid sequence of the mRNA.

18. The method according to any one of claims 1-17, wherein the probe also comprises a probe identity tag.

19. The method of claim 18, wherein in the probe, the target molecule binding domain is linked directly or indirectly with the probe identity tag.

20. The method according to any one of claims 18-19, wherein the probe identity tag comprises a unique molecular identification region.

21. The method of claim 20, wherein the unique molecular identification region comprises a single-stranded nucleic acid.

22. The method of claim 21, wherein the single-stranded nucleic acid comprises 1-20 nucleotides.

23. The method according to any one of claims 1-22, comprising contacting at least one target molecule in the sample with two or more probes.

24. The method of claim 23, wherein the two or more probes have respective distinct probe identity tags.

25. The method according to any one of claims 1-24, comprising contacting at least one target molecule in the sample with a first probe, and contacting the target molecule with a second probe different from the first probe.

26. The method of claim 25, wherein a 3' end of the first probe is directly or indirectly linked to a 5' end of the second probe.

27. The method according to any one of claims 25-26, wherein a 5' end of the first probe is directly or indirectly linked to a 3' end of the second probe.

28. The method according to any one of claims 25-27, wherein the 3' end of the first probe is linked to the 5' end of the second probe by a ligase.

29. The method according to any one of claims 25-28, wherein the 5' end of the first probe is linked to the 3' end of the second probe by a ligase.

30. The method according to any one of claims 25-29, wherein the first probe and the second probe form a single-stranded cyclic molecule.

31. The method according to any one of claims 25-30, wherein a nucleic acid sequence at the 3' end of the first probe is complementary to a nucleic acid sequence at the 3' end of the second probe, and a nucleic acid sequence of the first probe is complementary to a nucleic acid sequence at the 5' end of the second probe.

32. The method according to any one of claims 25-31, wherein the 5' end of the second probe is directly or indirectly linked to the 3' end of the second probe.

33. The method according to any one of claims 25-32, wherein the 5' end of the second probe is ligated to the 3' end of the second probe by a ligase.

34. The method according to any one of claims 28-33, wherein the ligase comprises a T4 ligase.

35. The method according to any one of claims 1-34, wherein the probes in contact with target molecules derived from the same sample are attached with the same sample-specific barcode.

36. The method according to any one of claims 1-35, wherein the target molecule in the sample is mRNA in a single cell, and the method does not comprise a reverse transcription step.

37. The method according to any one of claims 1-36, wherein the probe also comprises an amplification primer binding region.

38. The method according to any one of claims 1-37, wherein the probe also comprises an oligonucleotide adapter.

39. The method according to any one of claims 37-38, wherein the amplification primer binding region in the probe is directly or indirectly linked to the probe identity tag.

40. The method according to any one of claims 37-39, wherein the amplification primer binding region in the probe is directly linked to the probe identity tag.

41. The method according to any one of claims 23-40, wherein at least two probes of the two or more probes are capable of binding to different regions of the same target molecule in the sample.

42. The method according to any one of claims 1-41, wherein, after contacting the target molecule in the sample with the probe and before attaching the probe with the sample-specific barcode, providing a solid support attached with at least one PCR tag, wherein each of the PCR tags comprises a sample-specific barcode and a PCR handle sequence at the 3' end of the sample-specific barcode which is the same as the sequence at the 5' end of the probe, and providing a free primer which is complementary to the sequence at the 3' end of the probe.

43. The method of claim 42, further comprising co-dispensing the probe, the solid support attached with at least one PCR tag, and the primer into a discrete compartment, and attaching the probe with the sample-specific barcode.

44. The method according to any one of claims 42-43, wherein the PCR tag is releasably attached to the solid support.

45. The method according to any one of claims 42-44, comprising releasing the at least one PCR tag from the solid support, and co-dispensing the PCR tag, the primer, and the probe into the discrete compartment, enabling the probe to be attached with the sample-specific barcode.

46. The method according to any one of claims 42-45, wherein the PCR tag is directly or indirectly attached to the solid support through its 5' end.

47. The method according to any one of claims 43-46, wherein the discrete compartment further comprise a PCR reagent, and attaching the probe with the sample-specific barcode under the action of the PCR reagent so as to obtain a barcoded probe.

48. The method according to any one of claims 43-47, comprising co-dispensing the probe and two or more of the PCR tags, the probe and the primer into the discrete compartment, the two or more PCR tags being different, so that the barcoded probe comprises two or more sample-specific barcodes.

49. The method according to any one of claims 43-48, comprising co-dispensing the probe and three or more of the PCR tags, the probe and the primer into the discrete compartment, and the three or more PCR tags being different, so that the barcoded probe comprises three or more sample-specific barcodes.

50. The method according to any one of claims 1-49, wherein in the barcoded probe, the probe is located at a 3' end of the sample-specific barcode.

51. The method according to any one of claims 42-50, wherein the solid support is a bead.

52. The method of claim 51, wherein the bead is a magnetic bead.

53. The method according to any one of claims 43-52, wherein the discrete compartment are pores or microdroplets.

54. The method according to any one of claims 42-53, wherein the sample-specific barcode comprises a cell barcode, and the cell barcodes comprised in each PCR tag attached to the same solid support are the same.

55. The method of claim 54, wherein the cell barcode comprises at least two cell barcode segments spaced by a linker sequence.

56. The method according to any one of claims 42-55, comprising making the primer complementary to the sequence at the 3' end of the probe and extending to obtain a sequence complementary to a hybridization sequence.

57. The method according to any one of claims 42-56, comprising hybridizing a sequence complementary to the sequence at the 5' end of the probe to a PCR handle sequence in the PCR tag and attaching the probe with a sample-specific barcode to obtain a barcoded probe.

58. The method according to any one of claims 42-57, wherein the PCR tag further comprises an amplification primer recognition region.

59. The method of claim 58, wherein the amplification primer recognition region is a universal amplification primer recognition region.

60. The method according to any one of claims 42-59, wherein the barcoded probe comprises the sample-specific barcode and the probe.

61. The method according to any one of claims 42-60, wherein the sample-specific barcode is located at the 3' end of the probe.

62. The method according to any one of claims 43-61, further comprising, after obtaining the barcoded probe and before determining the composition of the barcoded probe, releasing the barcoded probe from the discrete compartment.

63. The method according to any one of claims 43-62, comprising, after releasing barcoded probe from the discrete compartment and before determining the composition of the barcoded probe, sequencing the barcoded probe to determine the composition of the barcoded probe.

64. The method according to claim 63, comprising, after determining the composition of the barcoded probe, determining the presence and/or content of the target molecule from the number of the barcoded probes.

65. The method according to any one of claims 1-41, comprising, after contacting the target molecule in the sample with the probe and before attaching the probe with the sample-specific barcode, providing a solid support attached with at least one oligonucleotide tag, wherein each of the oligonucleotide tags comprises a sample-specific barcode and a hybridization sequence at the 3' end of the sample-specific barcode.

66. The method of claim 65, wherein the probe comprises a first strand and a second strand, the first strand comprises a target molecule binding domain.

67. The method of claim 66, wherein the second strand comprises a second portion complementary to the oligonucleotide adapter in the probe, and the second strand comprises a first portion complementary to the hybridization sequence at the 3' end of the sample-specific barcode in the oligonucleotide tag, the first and second strands forming a partially double-stranded structure.

68. The method according to any one of claims 65-67, wherein the probe is linked to the solid support attached with at least one oligonucleotide tag in the discrete compartment to obtain the barcoded probe.

69. The method according to any one of claims 65-68, wherein the oligonucleotide tag is releasably attached to the solid support.

70. The method according to any one of claims 65-69, comprising releasing the at least one oligonucleotide tag from the solid support and linking with the probe to attach the probe with the sample-specific barcode.

71. The method according to any one of claims 65-70, wherein the oligonucleotide tag is directly or indirectly attached to the solid support through its 5' end.

72. The method according to any one of claims 68-71, wherein the discrete compartment further comprises a ligase, and the ligase enables the oligonucleotide tag to be linked with the probe.

73. The method of claim 72, wherein the ligase comprises a T4 ligase.

74. The method according to any one of claims 68-73, wherein, in the barcoded probe, the probe is located at the 3' end of the sample-specific barcode.

75. The method according to any one of claims 65-74, wherein the solid support is a bead.

76. The method of claim 75, wherein the bead is a magnetic bead.

77. The method according to any one of claims 68-76, wherein the discrete compartment are pores or microdroplets.

78. The method according to any one of claims 65-77, wherein the sample-specific barcode comprises a cell barcode, and the cell barcodes comprised in each oligonucleotide tag attached to the same solid support are the same.

79. The method of claim 78, wherein the cell barcode comprises at least two cell barcode segments spaced by a linker sequence.

80. The method according to any one of claims 66-79, comprising linking the hybridization sequence in the oligonucleotide tag to the first strand in the probe to produce the barcoded probe.

81. The method according to any one of claims 67-80, comprising hybridizing the first portion of the second strand in the probe with the hybridization sequence of the oligonucleotide tag, and linking the hybridization sequence of the oligonucleotide tag to the first strand in the probe to produce the barcoded probe.

82. The method according to any one of claims 65-81, wherein the oligonucleotide tag further comprises an amplification primer recognition region.

83. The method according to claim 82, wherein the amplification primer recognition region is a universal amplification primer recognition region.

84. The method according to any one of claims 68-83, wherein the barcoded probe comprises the sample-specific barcode and the probe.

85. The method according to any one of claims 65-84, wherein the sample-specific barcode is located at the 3' end of the probe.

86. The method according to any one of claims 68-85, further comprising, after obtaining the barcoded probe and before determining the composition of the barcoded probe, releasing the barcoded probe from the discrete compartment.

87. The method according to any one of claims 68-86, comprising, after releasing the barcoded probe from the discrete compartment and before determining the composition of the barcoded probe, sequencing the barcoded probe to determine the composition of the barcoded probe.

88. The method of claim 87, comprising, after determining the composition of the barcoded probe, determining the presence and/or content of the target molecule from the number of the barcoded probes.

89. The method according to any one of claims 1-41, comprising, after contacting the target molecule in the sample with the probe and before attaching the probe with the sample-specific barcode, providing the solid support attached with at least one oligonucleotide tag, wherein each of the oligonucleotide tags comprises the first strand and the second strand, and the first strand comprises the sample-specific barcode and the hybridization sequence at the 3' end of the sample-specific barcode, the second strand comprises the first portion complementary to the hybridization sequence of the first strand and the second portion complementary to the sequence of the oligonucleotide adapter in the probe, and the first and second strands forming a partially double-stranded structure, and the solid support attached with at least one oligonucleotide tag is linked with the probe in the discrete compartment to obtain a barcoded probe.

90. The method of claim 89, wherein the oligonucleotide tag is releasably attached to the solid support.

91. The method according to any one of claims 89-90, comprising releasing the at least one oligonucleotide tag from the solid support and linking with the probe to attach the probe with the sample-specific barcode.

92. The method according to any one of claims 89-91, wherein the oligonucleotide tag is directly or indirectly attached to the solid support through the 5' end of its first strand.

93. The method according to any one of claims 89-92, wherein the discrete compartment further comprises a ligase, and the ligase enables the oligonucleotide tag to be linked with the probe.

94. The method of claim 93, wherein the ligase comprises a T4 ligase.

95. The method according to any one of claims 89-94, wherein in the barcoded probe, the probe is located at the 3' end of the sample-specific barcode.

96. The method according to any one of claims 89-95, wherein the solid support is a bead.

97. The method of claim 96, wherein the bead is a magnetic bead.

98. The method according to any one of claims 89-97, wherein the discrete compartment are pores or microdroplets.

99. The method according to any one of claims 89-98, wherein the sample-specific barcode comprises a cell barcode, and the cell barcodes comprised in each oligonucleotide tag attached to the same solid support are the same.

100. The method of claim 99, wherein the cell barcode comprises at least two cell barcode segments spaced by a linker sequence.

101. The method according to any one of claims 89-100, comprising linking the hybridization sequence of the first strand of the oligonucleotide tag to the probe to produce the barcoded probe.

102. The method according to any one of claims 89-101, comprising hybridizing the second portion of the second strand of the oligonucleotide tag to the oligonucleotide adapter in the probe, and linking the hybridization sequence of the first strand of the oligonucleotide tag to the probe to produce the barcoded probe.

103. The method according to any one of claims 89-102, wherein the oligonucleotide tag further comprising an amplification primer recognition region.

104. The method of claim 103, wherein the amplification primer recognition region is a universal amplification primer recognition region.

105. The method according to any one of claims 89-104, wherein the barcoded probe comprises the sample-specific barcode and the probe.

106. The method according to any one of claims 89-105, wherein the sample-specific barcode is located at the 3' end of the probe.

107. The method according to any one of claims 89-106, further comprising, after obtaining the barcoded probe and before determining the composition of barcoded probe, releasing the barcoded probe from the discrete compartment.

108. The method according to any one of claims 89-107, comprising, after releasing the barcoded probe from the discrete compartment and before determining the composition of the barcoded probe, sequencing the barcoded probe to determine the composition of the barcoded probe.

109. The method of claim 108, comprising, after determining the composition of the barcoded probe, determining the presence and/or content of the target molecule from the number of barcoded probes.

110. The method according to any one of claims 1-10, wherein the target molecule comprises a protein, and the target molecule binding domain in the probe comprises an antigen binding portion that specifically recognizes the protein.

111. The method of claim 110, wherein the antigen binding portion comprises an antigen binding protein and/or an antigen binding nucleic acid molecule.

112. The method of claim 111, wherein the antigen binding protein comprises an antibody or an antigen binding fragment.

113. The method of claim 112, wherein the antigen binding fragment comprises Fab, Fab', F(ab)2, Fv fragment, F(ab')2, scFv, di-scFv, VHH, and/or dAb.

114. The method according to any one of claims 111-113, wherein the antigen binding nucleic acid molecule comprises a nucleic acid aptamer.

115. The method according to any one of claims 110-114, wherein the identity of the protein is known.

116. The method according to any one of claims 110-115, wherein the amino acid sequence of the protein is known.

117. The method according to any one of claims 112-116, wherein the amino acid sequence of the antibody or antigen binding fragment is known, and the antibody or antigen binding fragment specifically binds to the protein.

118. The method according to any one of claims 110-117, wherein the probe also comprises a probe identity tag.

119. The method of claim 118, wherein the target molecule binding domain is directly or indirectly linked to the probe identity tag.

120. The method according to any one of claims 118-119, wherein the probe identity tag characterizes the identity of the antibody or antigen binding fragment.

121. The method of any one of claims 110-120, comprising contacting the target molecule in the sample with two or more probes.

122. The method of claim 121, wherein the two or more probes have respective different probe identity tags.

123. The method according to any one of claims 110-122, wherein the probes in contact with target molecules derived from the same sample are attached with the same sample-specific barcode.

124. The method according to any one of claims 110-123, wherein the probe also comprises an amplification primer binding region.

125. The method according to any one of claims 110-124, wherein the probe also comprises an oligonucleotide adapter.

126. The method according to any one of claims 124-125, wherein the amplification primer binding region in the probe is directly or indirectly linked to the probe identity tag.

127. The method according to any one of claims 124-126, wherein the amplification primer binding region in the probe is directly linked to the probe identity tag.

128. The method according to any one of claims 110-127, wherein, after contacting the target molecule in the sample with the probe and before attaching the probe with the sample-specific barcode, providing a solid support attached with at least one oligonucleotide tag, wherein each of the oligonucleotide tags comprises the first strand and the second strand, and the first strand comprises the sample-specific barcode and the hybridization sequence at the 3' end of the sample-specific barcode, the second strand comprises the first portion complementary to the hybridization sequence of the first strand and the second portion complementary to the sequence of the oligonucleotide adapter in the probe, and the first and second strands forming a partially double-stranded structure, and the solid support attached with at least one oligonucleotide tag is linked with the probe in the discrete compartment to obtain a barcoded probe.

129. The method of claim 128, wherein the oligonucleotide tag is releasably attached to the solid support.

130. The method according to any one of claims 128-129, comprising releasing the at least one oligonucleotide tag from the solid support and linking with the probe to attach the probe with the sample-specific barcode.

131. The method according to any one of claims 128-130, wherein the oligonucleotide tag is directly or indirectly attached to the solid support through the 5' end of its first strand.

132. The method according to any one of claims 128-131, wherein the discrete compartment further comprises a ligase, and the ligase enables the oligonucleotide tag to be linked with the probe.

133. The method of claim 132, wherein the ligase comprises a T4 ligase.

134. The method according to any one of claims 128-133, wherein in the barcoded probe, the probe is located at the 3' end of the sample-specific barcode.

135. The method according to any one of claims 128-134, wherein the solid support is a bead.

136. The method of claim 135, wherein the bead is a magnetic bead.

137. The method according to any one of claims 128-136, wherein the discrete compartment are pores or microdroplets.

138. The method according to any one of claims 128-137, wherein the sample-specific barcode comprises a cell barcode, and the cell barcodes comprised in each oligonucleotide tag attached to the same solid support are the same.

139. The method of claim 138, wherein the cell barcode comprises at least two cell barcode segments spaced by a linker sequence.

140. The method according to any one of claims 128-139, comprising linking the hybridization sequence of the first strand of the oligonucleotide tag to the probe to produce the barcoded probe.

141. The method of any one of claims 128-140, comprising hybridizing the second portion of the second strand of the oligonucleotide tag to the oligonucleotide adapter, and linking the hybridization sequence of the first strand of the oligonucleotide tag to the probe to produce the barcoded probe.

142. The method according to any one of claims 128-141, wherein the oligonucleotide tag further comprises an amplification primer recognition region.

143. The method of claim 142, wherein the amplification primer recognition region is a universal amplification primer recognition region.

144. The method according to any one of claims 128-143, wherein the barcoded probe comprises the sample-specific barcode and the probe.

145. The method according to any one of claims 128-144, wherein the sample-specific barcode is located at the 3' end of the probe.

146. The method according to any one of claims 128-145, further comprising, after obtaining the barcoded probe and before determining the composition of the barcoded probe, releasing the barcoded probe from the discrete compartment.

147. The method of claim 146, comprising, after determining the composition of the barcoded probe, determining the presence and/or content of the target molecule from the number of the barcoded probe.

148. The method according to any one of claims 1-147, comprising, before contacting the target molecule in the sample with the probe, pre-treating the sample.

149. The method of claim 148, wherein the pretreatment comprises immobilizing the sample with an immobilizing agent selected from one or more of the group consisting of formaldehyde, paraformaldehyde, methanol, ethanol, acetone, glutaraldehyde, osmic acid, and potassium dichromate.

150. The method according to any one of claims 148-149, wherein the pretreatment comprises exposing the cell nucleus of the sample.

151. The method according to any one of claims 148-150, wherein the pretreatment comprises treating the sample with a detergent comprising Triton, NP-40, and/or digitonin.

152. The method according to any one of claims 1-151, wherein the target molecule comprises protein and mRNA.

153. The method according to any one of claims 1-152, wherein the target molecule binding domain specifically recognizes the protein and binds mRNA.

154. A combination, comprising: (1) two or more probes, the probes comprising a target molecule binding domain and a probe identity tag; (2) a plurality of solid supports, each of the solid supports being attached with at least one PCR tag, wherein each of the PCR tags comprises a sample-specific barcode and a PCR handle sequence at the 3' end of the sample-specific barcode, the PCR handle sequence being the same as the sequence at the 5' end of the probe, and providing a free primer and a PCR reagent, the primer being complementary to the sequence at the 3' end of the probe.

155. The combination of claim 154, wherein the (1) and (2) exist independently of each other.

156. The combination of claim 154, wherein the (1) and (2) exist as a mixture.

157. A kit for analyzing a sample, comprising the combination of any one of claims 154-156.

158. The kit of claim 157, comprising at least one of a nucleic acid amplifying agent, an immobilizing agent, a permeating agent, and a lysing agent.

159. The kit according to any one of claims 157-158, comprising an instruction for use.

160. The kit of claim 159, wherein the instruction for use comprises the following steps:
contacting at least one target molecule in a sample with at least one probe, the probe comprising a target molecule binding domain and a probe identity tag;
attaching the probe to a sample-specific barcode to obtain a barcoded probe; and
determining the composition of the barcoded probe, and determining the presence and/or content of the target molecule in the sample from the composition of the barcoded probe.

161. A combination comprising (1) two or more probes, the probes comprising a target molecule binding domain and a probe identity tag; (2) a plurality of solid supports, each of the solid supports being attached with at least one oligonucleotide tag, wherein each of the oligonucleotide tags comprises a first strand and a second strand, the first strand comprising a sample-specific barcode and a hybridization sequence at a 3' end of the sample-specific barcode, the second strand comprising a first portion complementary to a hybridization sequence of the first strand and a second portion complementary to the probe identity tag, and the first and second strands forming a partially double-stranded structure.

162. The combination of claim 161, wherein the (1) and (2) exist independently of each other.

163. The combination of claim 161, wherein the (1) and (2) exist as a mixture.

164. A kit for analyzing a sample, comprising the combination of any one of claims 161-163.

165. The kit of claim 164, comprising at least one of a nucleic acid amplifying agent, a linking agent, an immobilizing agent, a permeating agent, and a lysing agent.

166. The kit according to any one of claims 164-165, comprising an instruction for use.

167. The kit of claim 166, wherein the instruction for use comprises the following steps: (a) contacting at least one target molecule in a sample with at least one probe, the probe comprising a target molecule binding domain and a probe identity tag; (b) attaching the probe to a sample-specific barcode to obtain a barcoded probe; and (c) determining the composition of the barcoded probe, and determining the presence and/or content of the target molecule in the sample from the composition of the barcoded probe.
